(19) Europäisches Patentamt
European Patent Office
Office européen des brevets

(11) EP 2 781 536 B1

(12) **EUROPEAN PATENT SPECIFICATION**

(45) Date of publication and mention of the grant of the patent:
**17.08.2016 Bulletin 2016/33**

(21) Application number: **12850688.8**

(22) Date of filing: **19.11.2012**

(51) Int Cl.:
***C08G 81/00*** *(2006.01)* ***C08G 69/40*** *(2006.01)*
***C08G 69/42*** *(2006.01)* ***A61K 47/48*** *(2006.01)*

(86) International application number:
**PCT/JP2012/079897**

(87) International publication number:
**WO 2013/073697 (23.05.2013 Gazette 2013/21)**

(54) **BLOCK COPOLYMER HAVING PHENYLBOROBIC ACID GROUP INTRODUCED THEREIN, AND USE THEREOF**

BLOCKCOPOLYMER MIT INTEGRIERTER PHENYLBORSÄUREGRUPPE UND VERWENDUNG

COPOLYMÈRE À BLOCS DANS LEQUEL UN GROUPE ACIDE PHÉNYLBORONIQUE A ÉTÉ INTRODUIT, ET UTILISATION DE CE DERNIER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.11.2011 US 201161561022 P**

(43) Date of publication of application:
**24.09.2014 Bulletin 2014/39**

(73) Proprietors:
- **The University of Tokyo**
**Bunkyo-ku,**
**Tokyo 113-8654 (JP)**
- **National University Corporation**
**Tokyo Medical and Dental University**
**Bunkyo-ku**
**Tokyo 113-8510 (JP)**
- **NanoCarrier Co., Ltd.**
**226-39 Wakashiba**
**Kashiwa**
**Chiba 277-0871 (JP)**

(72) Inventors:
- **KATAOKA, Kazunori**
**Tokyo 113-8654 (JP)**
- **ISHII, Takehiko**
**Tokyo 113-8654 (JP)**
- **NAITO, Mitsuru**
**Tokyo 113-8654 (JP)**
- **MATSUMOTO, Akira**
**Tokyo 113-8510 (JP)**
- **KATO, Yasuki**
**Kashiwa**
**Chiba 277-0871 (JP)**

(74) Representative: **Müller-Boré & Partner Patentanwälte PartG mbB**
**Friedenheimer Brücke 21**
**80639 München (DE)**

(56) References cited:
**EP-A1- 2 522 679**
**WO-A1-2009/133647**
**JP-A- 2002 179 683**
**JP-A- 2002 179 683**
**JP-A- 2011 173 960**

- **MIYATA ET AL: "PEG-based block catiomers possessing DNA anchoring and endosomal escaping functions to form polyplex micelles with improved stability and high transfection efficacy", JOURNAL OF CONTROLLED RELEASE, ELSEVIER, AMSTERDAM, NL, vol. 122, no. 3, 18 September 2007 (2007-09-18), pages 252-260, XP022336556, ISSN: 0168-3659, DOI: 10.1016/J.JCONREL.2007.06.020**
- **NAKANOWATARI F ET AL: "Micelle formation from PEG-p(Lys) block copolymer with phenyl boronic acid moieties", POLYMER PREPRINTS. JAPAN, SOCIETY OF POLYMER SCIENCE, JP, vol. 48, no. 3, 12 May 1999 (1999-05-12), XP008173084,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- SINGHAL R P ET AL: "New ligands for boronate affinity chromatography", JOURNAL OF CHROMATOGRAPHY, ELSEVIER SCIENCE PUBLISHERS B.V, NL, vol. 543, 1 January 1991 (1991-01-01), pages 17-38, XP026514921, ISSN: 0021-9673, DOI: 10.1016/S0021-9673(01)95752-8 [retrieved on 1991-01-01]
- AKIRA MATSUMOTO ET AL: "Glucose-Responsive Polymer Bearing a Novel Phenylborate Derivative as a Glucose-Sensing Moiety Operating at Physiological pH Conditions", BIOMACROMOLECULES, vol. 4, no. 5, 19 August 2003 (2003-08-19) , pages 1410-1416, XP055105744, ISSN: 1525-7797, DOI: 10.1021/bm034139o
- FUMIKO NAKANOWATARI ET AL.: 'Micelle formation from PEG-p(Lys) block copolymer with phenyl boronic acid moieties' POLYMER PREPRINTS, JAPAN vol. 48, no. 3, 12 May 1999, page 572, XP008173084
- KAZUNORI KATAOKA: 'Cellular specific material and new drug delivery system' POLYFILE vol. 30, no. 350, April 1993, pages 27 - 31, XP008174127
- MITSURU NAITO ET AL.: 'Ribose to Kagyaku Ketsugosei o Yusuru Phenylboronic Acid o Riyo shita Kobunshi Micelle-gata siRNA Delivery Carrier no Sekkei to Kino Hyoka' POLYMER PREPRINTS, JAPAN vol. 61, no. 1, 15 May 2012, page 1642, XP008173086
- L.ZHAO ET AL.: 'Glucose-sensitive polypeptide micelles for self-regulated insulin release at physiological pH' J. MATER. CHEM. vol. 22, 2012, pages 12319 - 12328, XP055150110

SEQUENCE LISTING

<110> The University of Tokyo National University Corporation Tokyo Medical and Dental University NanoCarrier Co., Ltd.

<120> Blockcopolymer in which a phenyboronic acid group is introduced and use thereof

<130> T 3361EU - py

<140> EP12850688
<141> 19.11.2012

<150> US61/561,022
<151> 17.11.2011

<160> 6

<170> PatentIn version 3.5

<210> 1
<211> 21
<212> DNA
<213> Artificial Sequence

<220>
<223> sense strand of siRNA for human vascular endothelial growth factor including dT terminus

<400> 1
gaucucauca ggguacucct t 21

<210> 2
<211> 21
<212> DNA
<213> Artificial Sequence

<220>
<223> antisense strand of siRNA for human vascular endothelial growth factor including dT terminus

<400> 2
ggaguacccu gaugagauct t 21

<210> 3
<211> 21
<212> RNA
<213> Artificial Sequence

<220>
<223> sense strand of non-therapeutic siRNA

<400> 3
uucuccgaac gugucacguu u 21

<210> 4
<211> 21
<212> RNA

<220>
<223> sense strand of non-therapeutic siRNA

<400> 3
uucuccgaac gugucacguu u 21

<210> 4
<211> 21
<212> RNA

<213> Artificial Sequence

<220>
<223> antisense strand of non-therapeutic siRNA

<400> 4
acgugacacg uucggagaau u 21

<210> 5
<211> 21
<212> RNA
<213> Artificial Sequence

<220>
<223> sense strand of siRNA for firefly luciferase

<400> 5
cuuacgcuga cuacuucgau u 21

<210> 6
<211> 21
<212> RNA
<213> Artificial Sequence

<220>
<223> antisense strand of siRNA for firefly luciferase

<400> 6
ucgaaguacu cagcguaagu u 21

## Description

### Technical Field

The present invention relates to a block copolymer that has a phenylboronic acid group introduced, and a complex including the block copolymer and a drug.

### Background Art

Biological pharmaceuticals each including a biopolymer such as a protein or a nucleic acid are easily decomposed with an enzyme or eliminated by an immune system as compared to related-art pharmaceuticals each including a low-molecular-weight compound. The inventors of the present invention have developed a drug delivery system (DDS) using a polyamino acid-based block copolymer from the viewpoint of improving delivery of such biotechnology-based drugs to an affected area. An aim of the development is to provide a carrier that simultaneously achieves stability in blood (retention property of the biotechnology-based drugs) and drug-releasing property at an affected area.

Meanwhile, for a different purpose from the DDS development, the inventors of the present invention have further made basic studies on a biocompatible material applicable to a saccharide sensor or a saccharide-responsive actuator. For example, there has been developed, as the biocompatible material, a phenylboronic acid-based compound having a fluorinated phenyl ring (Patent Literature 1).

It should be noted that Patent Literature 2 is the related art relating to a reagent that is obtained by binding a nucleic acid to a polyamino acid-based derivative using a phenylboronic acid group and is applied to analyses of behaviors and structures of nucleic acids and nucleic acid-related substances.

### Citation List

### Patent Literature

[Patent Literature 1] JP 2011-140537 A
[Patent Literature 2] JP 2002-179683 A

### Summary of the Invention

### Technical Problem

It is a main object of the present invention to provide a carrier that simultaneously achieves stability of a biotechnology-based drug in blood and drug-releasing property of the drug at an affected area. It should be noted that Patent Literature 2 describes that application of a reagent that carries a nucleic acid-related substance is developed as a DDS. However, the DDS thus developed is merely a prodrug like an RNA mimic and easily forms precipitates. The technology described in Patent Literature 2 is intended to provide a DDS different from the DDS developed by the inventors of the present invention, and cannot be easily applied as a carrier excellent in stability in blood.

### Solution to Problem

The inventors have found that a biocompatible material created for a purpose different from the development of the DDS can significantly improve stability in blood of a biotechnology-based drug including a polyamino acid-based block copolymer, and the present invention has been completed. That is, according to one aspect of the present invention, there is provided a block copolymer including a polyamino acid chain segment and a hydrophilic polymer chain segment, in which the polyamino acid chain segment includes an amino acid residue having a cationic group in a side chain and an amino acid residue having a substituted phenylboronic acid group in a side chain, the substituted phenylboronic acid group having a phenyl ring in which at least one hydrogen atom is substituted to have a pKa of less than 7.5; such as further described in the claims.

According to another aspect of the present invention, there is provided a complex. The complex is a complex including the block copolymer and a nucleic acid.

Advantageous Effects of Invention

According to one embodiment of the present invention, there is provided a carrier capable of simultaneously achieving stability of a biotechnology-based drug in blood and releasing property of the drug in a target cell.

Brief Description of Drawings

FIG. 1 is a graph showing a relationship between FPBA group contents and complex stability.
FIG. 2 is a graph showing the results of a toxicity test of block copolymers.
FIG. 3 is a graph showing the results of a toxicity test of complexes.
FIG. 4 is a graph showing pH stability of a complex.
FIG. 5 is a graph showing siRNA-releasing property of complexes.
FIG. 6 is a graph showing an ability to take up a complex into cells.
FIG. 7 is a graph showing retentivity of a complex in blood.
FIG. 8 is a graph showing anticancer activities of complexes.

Description of Embodiments

[A. Block copolymer]

Ablock copolymer of the present invention includes a polyamino acid chain segment and a hydrophilic polymer chain segment. The polyamino acid chain segment includes an amino acid residue having a cationic group in a side chain (hereinafter sometimes referred to as "cationic amino acid residue") and an amino acid residue having a substituted phenylboronic acid group (hereinafter sometimes referred to as "substituted PBA group") in a side chain, the substituted phenylboronic acid group having a phenyl ring in which at least one hydrogen atom is substituted by a halogen or a nitro group to have a pKa of less than 7.5 (hereinafter sometimes referred to as "substituted PBA group-containing amino acid residue"). In the context, the cationic amino acid residue may be different from or the same as the substituted PBA group-containing amino acid residue. Specifically, the polyamino acid chain segment may include a cationic amino acid residue free of the substituted PBA group in a side chain and a substituted PBA group-containing amino acid residue free of the cationic group in a side chain. Alternatively, instead of one or both of the amino acid residues or in addition to the amino acid residues, the polyamino acid chain segment may include an amino acid residue having both the cationic group and the substituted PBA group in a side chain.

From the viewpoint of compatibility with a body environment typified by blood (less than pH 7.5), in the substituted PBA group, at least one hydrogen atom of a phenyl ring constituting a phenylboronic acid group is substituted by a halogen or a nitro group so that the substituted phenylboronic acid group has a pKa of less than 7.5. The number of the hydrogen atoms substituted is 1, 2, 3, or 4. When only one hydrogen atom is substituted, the substituent and $B(OH)_2$ may be introduced at any one of ortho, meta, and para positions. Examples of the substituent include a halogen such as fluorine, chlorine, or bromine; and a nitro group. Of those, from the viewpoint of enhancing hydrophilicity of the block copolymer and the viewpoint of adjusting the pKa to less than 7.5, the substituted PBA group is preferably a fluorinated phenylboronic acid group represented by the following formula (I) (hereinafter sometimes referred to as "PBA group"). The pKa of the substituted PBA group is specified from a substituted PBA group-containing amino acid synthesized as a monomer. The lower limit of the pKa of the substituted PBA group is not particularly limited, and may be 2 or 3, for example.

[Chem. 1]

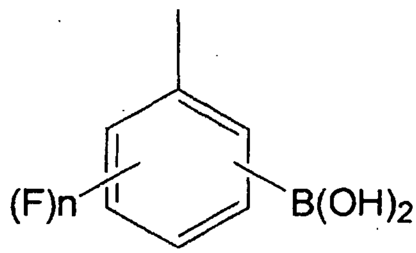

(I)

(In the formula (I): F's are present independently; n represents 1, 2, 3, or 4; and when n represents 1, F and $B(OH)_2$ may be introduced at any one of ortho, meta, and para positions.)

The block copolymer of the present invention has a cationic group in a side chain moiety of the polyamino acid chain segment, and hence can associate with a biopolymer to form a complex, for example, a polyion complex (PIC).

Inaddition, the block copolymer of the present invention has the substituted PBA group in a side chain moiety of the polyamino acid chain segment, and hence may exhibit the following effects. Firstly, hydrophobicity of the polyamino acid chain segment is enhanced, and hence a hydrophobic interaction as well as an electrostatic interaction is suitably caused among a plurality of block copolymers of the present invention in an aqueous solvent. As a result, the association force among the polymers is enhanced, and hence the block copolymer of the present invention may form a very stable polymer micelle in an aqueous solvent. It should be noted that the block copolymer of the present invention in the polymer micelle is estimated to be arranged in a radial fashion so that the polyamino acid chain segment faces the inside, and the hydrophilic polymer chain segment faces the outside. Examples of the aqueous solvent include water, physiological saline, aqueous buffers such as a phosphate buffer, a carbonate buffer, a borate buffer, and an acetate buffer.

Secondly, a phenylboronic acid compound has a reversible covalent binding ability for a molecule having a 1, 2-diol (cis-diol) or a 1, 3 -diol in an aqueous solvent. Therefore, the block copolymer of the present invention can be bound strongly to a biopolymer having a 1,2-diol or the like (for example, siRNA) in an aqueous solvent via a covalent bond to the substituted PBA group as well as an electrostatic bond to the cationic group, resulting in the formation of a stable complex (for example, a polymer micelle including the biopolymer encapsulated therein). In particular, the block copolymer of the present invention can be bound to siRNA having cis-diols in the 3'-terminal ribose of the both double strands at the two 3'-terminals, and hence may form a very stable complex.

Thirdly, the phenylboronic acid usually has a pKa of about from 8 to 9, and hence has the maximum covalent binding ability for the molecule having a 1,2-diol or the like at pH around the pKa. Therefore, it has been considered that use of the phenylboronic acid is difficult in principle under a body environment of less than pH 7.5. However, in the substituted PBA group to be introduced into the block copolymer of the present invention, a hydrogen atom of the phenyl ring is substituted so that the group has a pKa around the physiological pH (exhibits preferably a pKa of less than 8, more preferably a pKa of less than 7.5), and hence the substituted PBA group can suitably exhibit the binding ability under the body environment.

Fourthly, the substituted PBA group is highly hydrophobized under an environment of pH equal to or less than the pKa. Therefore, when the pKa is appropriately controlled, it is possible to enhance both a bond to the molecule having a 1,2-diol or the like and a hydrophobic interaction among the block copolymers under the body environment. As a result, a very stable complex formed with the molecules can be obtained.

[A-1. Polyamino acid chain segment]

The polyamino acid chain segment includes a cationic amino acid residue having a cationic group in a side chain and a substituted PBA group-containing amino acid residue having a substituted PBA group in a side chain.

The cationic amino acid residue is preferably a cationic amino acid residue having an amino group in a side chain. When the cationic amino acid residue has the amino group in the side chain, the amino group can coordinate with boron of the substituted PBA group in an aqueous solvent. As a result, hydrophobization of the block copolymer due to introduction of the substituted PBA group can be avoided to maintain high hydrophilicity. It should be noted that even in a state where the amino group coordinates with boron, the binding ability of the substituted PBA group for the molecule having a cis-diol or the like can be maintained.

As an amino acid from which the cationic amino acid residue having an amino group in a side chain is derived, there are given, for example: a basic amino acid such as lysine, ornithine, arginine, homoarginine, or histidine; and an amino acid derivative obtained by introducing any appropriate amine compound into an acidic amino acid. Of those, preferred are lysine and an amino acid derivative obtained by substituting the -OH moiety of a carboxyl group (-C(=O)OH) of an acidic amino acid by any one of the groups of the following formulas (i) to (iv), more preferred are lysine and an amino acid derivative obtained by substituting the -OH moiety of a carboxyl group (-C(=O)OH) at the $\alpha$-position or $\beta$-position of aspartic acid or at the $\alpha$-position or $\gamma$-position of glutamic acid by any one of the groups of the following formulas (i) to (iv), and still more preferred are lysine and an amino acid derivative obtained by substituting the -OH moiety of a carboxyl group (-C(=O)OH) at the $\alpha$-position or $\beta$-position of aspartic acid or at the $\alpha$-position or $\gamma$-position of glutamic acid by the group of the following formula (i) :

- $NH-(CH_2)_{p1}-[NH-(CH_2)_{q1}-]_{r1}NH_2$ (i) ;
- $NH-(CH2)_{p2}-N[-(CH_2)_{q2}-NH_2]_2$ (ii) ;
- $NH-(CH_2)_{p3}-N\{[-(CH_2)_{q3}-NH_2][-(CH_2)_{q4}-NH-]_{r2}H\}$ (iii) ; and
- $NH-(CH_2)_{p4}-N\{-(CH_2)_{q5}-N[-(CH_2)_{q6}-NH_2]_2\}_2$ (iv)

in the formulas (i) to (iv) : p1 to p4, q1 to q6, and r1 and r2 each independently represent an integer of 1 to 5.

In the formulas (i) to (iv), p1 to p4 and q1 to q6 each independently represent preferably 2 or 3, more preferably 2. In addition, r1 and r2 each independently represent preferably an integer of 1 to 3.

When the cationic amino acid residue is a lysine residue, there are advantages in that a polyamino acid chain is easily synthesized and the resultant block copolymer is very excellent in biocompatibility. On the other hand, it has been revealed that when the cationic amino acid residue is an amino acid residue obtained by substituting the -OH moiety of a carboxyl group (-C(=O)OH) of an acidic amino acid by any one of the groups of the formulas (i) to (iv), the residues have a plurality of different amine functional groups and hence show a plurality of stages of pKa's, and a plurality of amine functional groups are partially protonated under a physiological condition at pH 7.4 and have a small damage on cells. In addition, there is an advantage in that an interaction with a nucleic acid or the like enables suitable formation of a complex such as a PIC. Further, when the complex thus formed is integrated into an endosome (pH 5.5), the protonation of the polyamino acid chain segment further proceeds due to decrease of environmental pH, and endosomal escape may be promoted based on a buffer effect (or a proton sponge effect) or enhanced membrane-damaging activity. As a result, it is possible to improve drug delivery efficiency to a cytoplasm.

The substituted PBA group is introduced into the side chain of the substituted PBA group-containing amino acid residue via an amide bond, a carbamoyl bond, an alkyl bond, an ether bond, an ester bond, a thioester bond, a thioether bond, a sulfonamide bond, a urethane bond, a sulfonyl bond, a thymine bond, a urea bond, or a thiourea bond.

As an amino acid residue into which the substituted PBA group introduced, any appropriate amino acid residue may be selected as long as the substituted PBA group is introduced via the linking group. From the viewpoint of ease of synthesis, the substituted PBA group is preferably introduced into the cationic amino acid residue having an amino group in a side chain. Specifically, the substituted PBA group may be introduced into the cationic amino acid residue having an amino group in a side chain via an amide bond formed by a reaction of the amino group with carboxyphenylboronic acid in which at least one hydrogen atom of a phenyl ring is substituted or an ester thereof. In the context, only one substituted PBA group or a plurality of substituted PBA groups may be introduced into a cationic amino acid residue having a plurality of amino groups in a side chain. When only one substituted PBA group is introduced, the substituted PBA group-containing amino acid residue after the introduction has both of the amino group and the substituted PBA group in the side chain, and hence is also a cationic amino acid residue. Therefore, in the present invention, apolyamino acid chain segment containing only such amino acid residue is also understood to include both of the cationic amino acid residue and the substituted PBA group-containing amino acid residue. However, in determining the sum of the number of the cationic amino acid residues and the number of the substituted PBA group-containing amino acid residues in the polyamino acid chain segment containing such amino acid residue, such amino acid residues are counted as any one of the cationic amino acid residues and the substituted PBA group-containing amino acid residues.

The polyamino acid chain segment may further include an amino acid residue having a hydrophobic group in a side chain (hereinafter sometimes referred to as "hydrophobic amino acid residue") as well as the cationic amino acid residue and the substituted PBA group-containing amino acid residue. When the polyamino acid chain segment includes the hydrophobic amino acid residue, a hydrophobic interaction among the block copolymers of the present invention increases in an aqueous solvent. As a result, a more stable polymer micelle may be formed. In addition, the hydrophobic amino acid residue sticks into a hydrophobic moiety on a cell membrane and can function as an anchor for fixing the polymer micelle on a cell membrane. Therefore, when a biopolymer such as a nucleic acid is encapsulated in the polymer micelle, a ratio of the biopolymer introduced into cells can be improved.

An amino acid from which the hydrophobic amino acid residue is derived is preferably exemplified by an amino acid having a solubility in 100 g of water at 25°C of 5 g or less, more preferably 4 g or less. Examples of such amino acid include a non-polar natural amino acid such as leucine, isoleucine, phenylalanine, methionine, or tryptophan and a hydrophobic derivative of an amino acid that has a hydrophobic group introduced into a side chain. A preferred example of the hydrophobic derivative of the amino acid is a derivative that has a hydrophobic group introduced into a side chain of an acidic amino acid such as aspartic acid or glutamic acid.

The hydrophobic group to be introduced may be preferably exemplified by a saturated or unsaturated linear or branched aliphatic hydrocarbon group having 6 to 27 carbon atoms, an aromatic hydrocarbon group having 6 to 27 carbon atoms, or a steryl group.

The saturated linear or branched aliphatic hydrocarbon group having 6 to 27 carbon atoms is exemplified by a pentacosyl group, a hexacosyl group, or a heptacosyl group as well as the alkyl group having 6 to 27 carbon atoms.

An example of the unsaturated linear or branched aliphatic hydrocarbon group having 6 to 27 carbon atoms is a group in which 1 to 5 carbon-carbon single bonds in a chain of the alkyl group having 6 to 27 carbon atoms are replaced by carbon-carbon double bonds. As an unsaturated fatty acid having such group, there are given, for example, lauric acid (or dodecanoic acid), myristic acid (or tetradecanoic acid), palmitic acid (or hexadecanoic acid), palmitoleic acid (or 9-hexadecenoic acid), stearic acid (or octadecanoic acid), oleic acid, linoleic acid, linolenic acid, eleostearic acid (or 9,11,13-octadecatrienoic acid), arachidic acid, arachidonic acid, behenic acid, lignoceric acid, nervonic acid, cerotic acid, and montanic acid.

Examples of the aromatic hydrocarbon group having 6 to 27 carbon atoms include an aryl group and an aralkyl group. Preferred specific examples of those groups include a phenyl group, a naphthyl group, a tolyl group, a xylyl group, a benzyl group, and a phenethyl group.

A sterol from which the steryl group is derived means a natural, semisynthetic, or synthetic compound based on a cyclopentanone hydrophenanthrene ring ($C_{17}H_{28}$) and derivatives thereof. For example, a natural sterol is exemplified by, but not limited to, cholesterol, cholestanol, dihydrocholesterol, cholic acid, campesterol, or sitosterol. Semisynthetic or synthetic compounds thereof may be, for example, synthetic precursors of those natural products (as necessary, encompassing a compound in which part or all of, if present, certain functional groups, hydroxy groups have been protected with a hydroxy protective group known in the art, or a compound in which a carboxyl group has been protected with a carboxyl protective group). Further, the sterol derivative means that, for example, without adversely affecting the object of the present invention, a $C_{1-12}$ alkyl group, a halogen atom such as chlorine, bromine, or fluorine may be introduced into a cyclopentanone hydrophenanthrene ring, and the ring system may be saturated or partially unsaturated. The sterol from which the steryl group is derived is preferably a sterol originating from an animal or vegetable oil such as cholesterol, cholestanol, dihydrocholesterol, cholic acid, campesterol, or sitosterol, more preferably cholesterol, cholestanol, or dihydrocholesterol, particularly preferably cholesterol.

The polyamino acid chain segment may include, as each of the cationic amino acid residue, the substituted PBA group-containing amino acid residue, and the hydrophobic amino acid residue, one kind of amino acid residue or two or more kinds of amino acid residues. In addition, the cationic amino acid residue, substituted PBA group-containing amino acid residue, and hydrophobic amino acid residue in the polyamino acid chain segment are bound to each other in any suitable order, and a random structure or a block structure may be applicable.

The numbers of the cationic amino acid residues, substituted PBA group-containing amino acid residues, and hydrophobic amino acid residues contained in the polyamino acid chain segment may be appropriately adjusted depending on the kind of each of the amino acid residues, the application of the block copolymer, or the like. From the viewpoint of improving retention property (stability in blood) of the biopolymer with additional certainty, the number of each of the amino acid residues is preferably adjusted so that the following relational expression is satisfied. It should be noted that when a plurality of cationic groups are present in a repeating unit (amino acid residue) constituting the polyamino acid chain segment, a plurality of substituted PBA groups may be introduced into one repeating unit. Therefore, the total number of the substituted PBA groups in the polyamino acid chain segment may exceed the sum of the number of the cationic amino acid residues that have no substituted PBA group introduced and the number of the amino acid residues that have the substituted PBA group introduced (substituted PBA group-containing amino acid residue). In addition, in an analysis by a general-purpose structural analysis device, it may be difficult to distinguish whether a plurality of the substituted PBA groups are introduced into one repeating unit or separately introduced into a plurality of repeating units. Therefore, in the relational expression, for convenience sake, the number of the cationic amino acid residues free of the substituted PBA group may be defined as a value determined by subtracting the number of the substituted PBA groups (measured value) from the sum of the number of the cationic amino acid residues and the number of the substituted PBA group-containing amino acid residues (theoretical value) (however, the lower limit is defined as 0). It should be noted that the upper limit of the following relational expression is not particularly limited, and may be, for example, 40, 38, 35, or 25. When the value of the following relational expression is 14 or more, or 15 or more, the stability in blood is improved with more certainty.

[Math. 1]

$$\sqrt{\text{Number of cationic amino acid residues free of substituted phenylboronic acid group in polyamino acid chain segment}} + 2\sqrt{\text{Number of substituted phenylboronic acid groups in polyamino acid chain segment}} \geqq 12.0$$

[A-2. Hydrophilic polymer chain segment]

The hydrophilic polymer chain segment may be formed of any appropriate hydrophilic polymer. Examples of the hydrophilic polymer include poly(ethylene glycol), polysaccharide, poly(vinylpyrrolidone), poly(vinyl alcohol), poly(acrylamide), poly (acrylic acid), poly(methacrylamide), poly (methacrylic acid), poly (methacrylic acid ester), poly (acrylic acid ester), polyamino acid, poly(malic acid), and derivatives thereof. Specific examples of the polysaccharide include starch, dextran, fructan, and galactan. Of those, poly(ethylene glycol) may be preferably used because terminal-reactive polyethylene glycols having a variety of functional groups at their terminus are commercially available, polyethylene glycols having a variety of molecular weights are commercially available, and these polyethylene glycols are readily available.

[A-3. Specific examples of block copolymer]

Specific examples of a block copolymer in a particularly preferred embodiment of the present invention are represented by the formulas (1) to (4). In the block copolymers of the formulas (1) to (4), the substituted PBA group is introduced into a side chain of a cationic amino acid residue.

[Chem. 2]

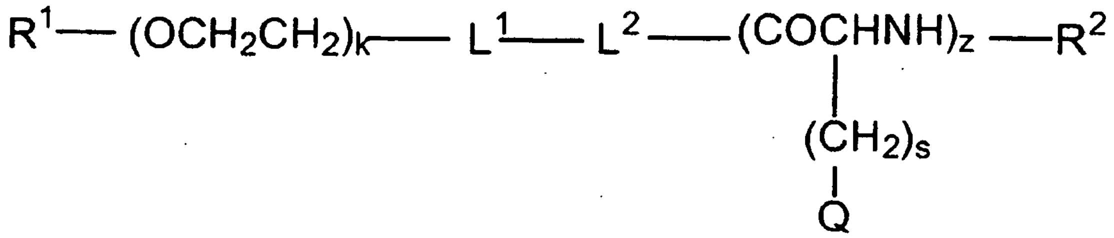

(1)

[Chem. 3]

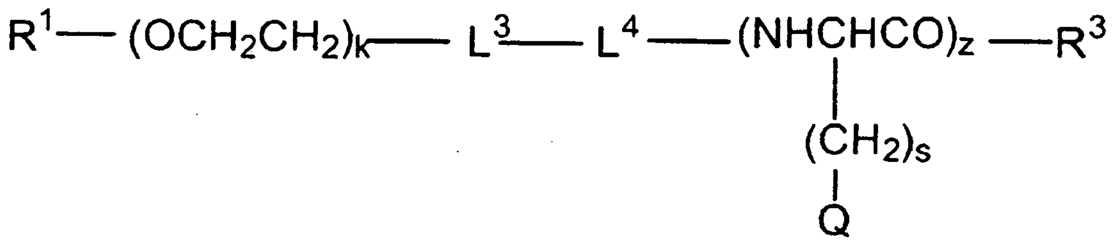

(2)

[Chem. 4]

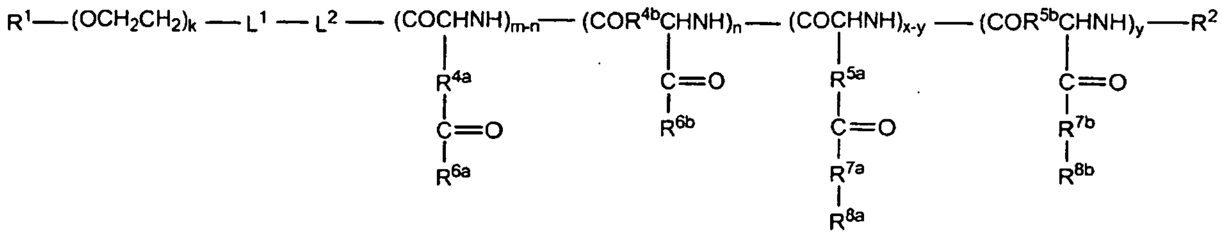

(3)

[Chem. 5]

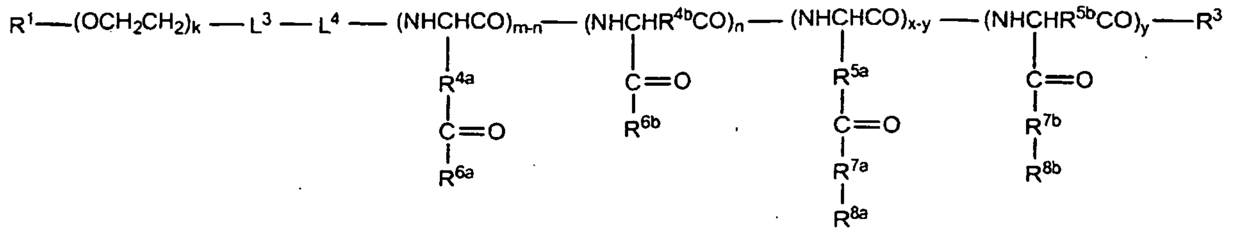

(4)

(In the formulas (1) to (4) : the group $R^1$ represents a hydrogen atom or an unsubstituted or substituted linear or branched alkyl group having 1 to 12 carbon atoms; the group $R^2$ represents a hydrogen atom, an unsubstituted or substituted linear or branched alkyl group having 1 to 12 carbon atoms, or an unsubstituted or substituted linear or branched alkylcarbonyl group having 1 to 24 carbon atoms; the group $R^3$ represents a hydroxyl group, an unsubstituted or substituted linear or branched alkyloxy group having 1 to 12 carbon atoms, an unsubstituted or substituted linear or branched alkenyloxy group having 2 to 12 carbon atoms, an unsubstituted or substituted linear or branched alkynyloxy group having 2 to 12 carbon atoms, or an unsubstituted or substituted linear or branched alkyl-substituted imino group having 1 to 12 carbon atoms; the groups $R^{4a}$, $R^{4b}$, $R^{5a}$, and $R^{5b}$ each independently represent a methylene group or an ethylene group; the groups $R^{6a}$ and $R^{6b}$ each independently represent a group selected from groups of the above-described formulas (i) to (iv); the groups $R^{7a}$ and $R^{7b}$ each independently represent -O- or -NH- ; the groups $R^{8a}$ and $R^{8b}$ each independently represent a saturated or unsaturated linear or branched aliphatic hydrocarbon group having 6 to 27 carbon atoms, an aromatic hydrocarbon group having 6 to 27 carbon atoms, or a steryl group; the group Q represents $-NH_2$, $-NHC(=NH)NH_2$, or a group represented by the following formula (II) ;

[Chem. 6]

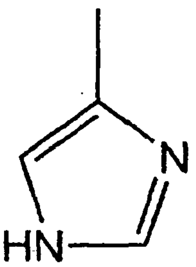

(II)

$L^1$ and $L^3$ each independently represent -S-S- or a valence bond; $L^2$ represents -NH-, -O- , $-O(CH_2)_{p1}-NH-$, or $-L^{2a}-(CH_2)_{q1}-L^{2b}-$, where p1 and q1 each independently represent an integer of 1 to 5, $L^{2a}$ represents OCO, OCONH, NHCO, NHCOO, NHCONH, CONH, or COO, and $L^{2b}$ represents NH or O; $L^4$ represents $-OCO-(CH_2)_{p2}-CO-$, $-NH-CO-(CH_2)_{p3}-CO-$, or $-L^{4a}-(CH_2)_{q2}-CO-$, where p2 , p3 , and q2 each independently represent an integer of 1 to 5, $L^{4a}$ represents OCONH, $-CH_2NHCO-$, NHCOO, NHCONH, CONH, or COO; k represents an integer of 30 to 20,000; s represents an integer of 1 to 6; m represents an integer of 1 to 300; n represents an integer of 0 to m; and x represents an integer of 0 to 80; y represents an integer of 0 to x; z represents an integer of 2 to 300; provided that when the total number of primary amino groups and secondary amino groups included in z pieces of the group Q or the total number of primary amino groups and secondary amino groups included in (m-n) pieces of the group $R^{6a}$ and n pieces of the group $R^{6b}$ is defined as w, 1 or more and less than w pieces of hydrogen atoms of the amino groups are substituted by an acyl group having a substituted PBA group (for example, an FPBA group represented by the formula (I)).)

In the formulas (1) to (4), both of $L^1$ and $L^2$ and both of $L^3$ and $L^4$ each need to be combined together so as to form one linking group. For example, when $L^2$ represents -NH-, $L^1$ does not represent -S-S- but represents a valence bond.

In the formulas (1) to (4), k, which represents the number of repetitions of ethylene glycol (or oxyethylene), represents an integer of 30 to 20,000, preferably 40 to 2,000, more preferably 50 to 1,500.

An alkyl moiety in the linear or branched alkyloxy group, alkyl-substituted imino group, and alkyl group having 1 to 12 carbon atoms, which are defined by the groups $R^1$ to $R^3$, may be, for example, a methyl group, an ethyl group, an n-propyl group, an isopropyl group, an n-butyl group, an sec-butyl group, a tert-butyl group, an n-hexyl group, a decyl group, and an undecyl group. An alkenyl or alkynyl moiety in the linear or branched alkenyloxy group having 2 to 12 carbon atoms or the linear or branched alkynyloxy group having 2 to 12 carbon atoms may be exemplified by an alkenyl

or alkynyl moiety including a double bond or a triple bond in the alkyl group having 2 or more carbon atoms as exemplified above.

For such group or moiety, a substituent in a "substituted" case may be exemplified by, but not limited to, a $C_{1-6}$ alkoxy group, an aryloxy group, an aryl $C_{1-3}$ oxy group, a cyano group, a carboxyl group, an amino group, a $C_{1-6}$ alkoxycarbonyl group, a $C_{2-7}$ acylamide group, a tri-$C_{1-6}$ alkyl siloxy group, a siloxy group, or a silylamino group, or may be exemplified by an acetalized formyl group, a formyl group, or a halogen atom such as chlorine or fluorine. In this context, for example, the expression "$C_{1-6}$" means 1 to 6 carbon atoms and is used with the same meaning in the following description. In addition, an unsubstituted or substituted linear or branched alkyl moiety having 1 to 12 carbon atoms in the unsubstituted or substituted linear or branched alkylcarbonyl group having 1 to 24 carbon atoms may be selected with reference to the above-described examples, and an alkyl moiety having 13 or more carbon atoms may be, for example, a tridecyl group, a tetradecyl group, a pentadecyl group, a nonadecyl group, a docosanyl group, or a tetracosyl group.

In another embodiment, a substituent for the group $R^1$ may be a group containing a target binding site. The introduction of the target binding site into the terminus of a polymer can improve drug (biopolymer) delivery to a desired target site. The group containing the target binding site may be any appropriate group as long as the group has targeting property or functionality for a target tissue. For example, the group may be a group originating from a physiologically active substance such as an antibody or a fragment thereof, or another protein having functionality or targeting property, a peptide, an aptamer, a sugar such as lactose, or folic acid, and a derivative thereof.

An example of the group $R^1$ substituted by a group including the target binding site is represented by the following formula (III):

[Chem. 7]

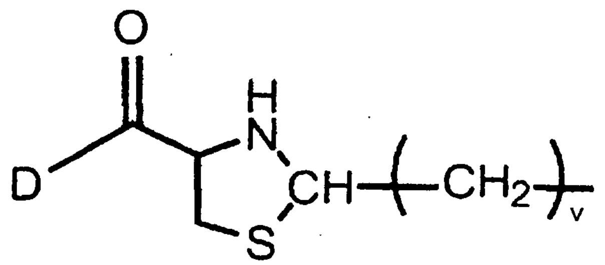

(III)

where: v represents an integer of 1 to 5; and D represents the target binding site.

D represents preferably a peptide having a weight average molecular weight of 50 to 20,000, more preferably a peptide having a weight average molecular weight of 100 to 10,000, still more preferably a peptide having a weight average molecular weight of 150 to 3,000.

Further, D represents preferably a peptide having 1 to 200 amino acid residues, more preferably a peptide having 1 to 100 amino acid residues, still more preferably a peptide having 1 to 30 amino acid residues.

Examples of the peptide include peptides capable of specifically binding to integrin, which is involved in angiogenesis, intimal thickening, and malignant tumor growth, and specific examples thereof include RGD peptides. By using an RGD peptide as the target binding site, particles, which are capable of specifically recognizing a diseased portion, and pharmaceutical compositions using the particles are obtainable. The RGD peptides as used herein refer to peptides that include an arginine-glycine-aspartic acid (RGD) sequence. The RGD peptide is preferably a cyclic RGD (cRGD) peptide. Specifically, D may be represented by the following formula (IV) :

[Chem. 8]

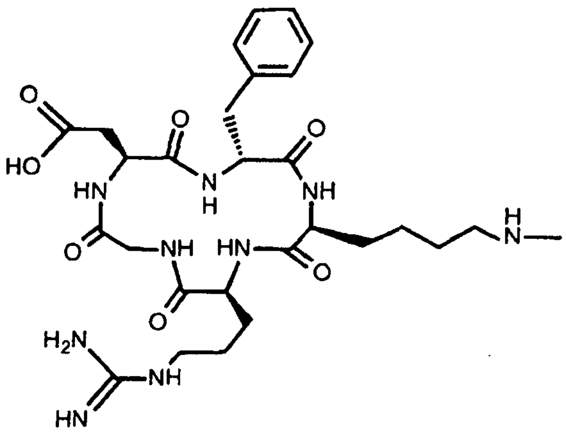

(IV)

In the formulas (1) to (4), the groups of the formulas (i) to (iv) defined by the groups $R^{6a}$ and $R^{6b}$ and the hydrocarbon group or steryl group defined by the groups $R^{8a}$ and $R^{8b}$ are as mentioned above. The groups Q, $R^{6a}$, $R^{6b}$, $R^{8a}$, and $R^{8b}$ selected for all of repeating units may be the same as or different from one another. Further, s represents 1, 3, or 4, for example.

In the formula (3) or (4), when both of the groups $R^{4a}$ and $R^{4b}$ each represent an ethylene group, a polyamino acid in which n represents an integer of 0 or a polyamino acid in which m-n represents an integer of 0 is typically represented. The former represents, for example, poly-$\alpha$-glutamic acid, which is obtained by the polymerization of an N-carboxylic anhydride of glutamic acid $\gamma$-benzyl ester, and the latter represents, for example, poly-$\gamma$-glutamic acid, which is produced by a bacterial strain of the genus Bacillus bacteria such as *Bacillus natto*. Meanwhile, when both the groups of $R^{4a}$ and $R^{4b}$ each represent a methylene group, it is understood that the respective repeating units having those groups may coexist with each other. The same holds true for the groups $R^{5a}$ and $R^{5b}$. It is preferred that the groups $R^{4a}$ and $R^{4b}$ each represent a methylene group and the groups $R^{5a}$ and $R^{5b}$ each represent an ethylene group from the viewpoint of production efficiency.

From the viewpoint of stability of the polymer micelle formed, the total number z of the cationic amino acid residues and substituted PBA group-containing amino acid residues included in the block copolymer of the formula (1) or (2) is an integer of 1 to 300, preferably 20 to 300, more preferably 30 to 200, still more preferably 40 to 150.

From the viewpoint of stability of the polymer micelle formed, the total number m of the cationic amino acid residues and substituted PBA group-containing amino acid residues included in the block copolymer of the formula (3) or (4) is an integer of 1 to 300, preferably 1 to 200, more preferably 1 to 150. When the block copolymer includes a hydrophobic amino acid residue (that is, when x represents any number other than 0), the number of the cationic amino acid residues may be, for example, an integer of 1 to 20, preferably 1 to 15, more preferably 1 to 10, still more preferably 1 to 5. Such block copolymer allows release of a biopolymer typified by a nucleic acid more suitably.

The number x of the hydrophobic amino acid residues that may be included in the block copolymer of the formula (3) or (4) may be appropriately adjusted depending on the kind or number of the cationic amino acid residues, the application of the block copolymer, or the like. The number x of the hydrophobic amino acid residues is an integer of preferably 1 to 80, more preferably 5 to 70, still more preferably 10 to 60.

In the block copolymers of the formulas (1) to (4), the number of the substituted PBA group-containing amino acid residues may be appropriately adjusted depending on the kind or number of the cationic amino acid residues, the application of the block copolymer, or the like. In the block copolymers, in particular, in the block copolymers of the formulas (1) and (2), the substituted PBA group is preferably introduced into the side chain of the cationic amino acid residue so that the following relationship is satisfied. When such relationship is satisfied, the retention property of the biopolymer by the block copolymer (stability in blood) is improved with more certainty. It should be noted that the upper limit of the following relational expression is not particularly limited, and may be 40, 38, 35, or 25, for example. When the value of the following relational expression is 14 or more, or 15 or more, the stability in blood is improved with more certainty.

[Math. 2]

$$\sqrt{\text{Number of cationic amino acid residues free of substituted phenylboronic acid group in polyamino acid chain segment}} + 2\sqrt{\text{Number of substituted phenylboronic acid groups in polyamino acid chain segment}} \geqq 12.0$$

[A-4. Preparation method for block copolymer]

The block copolymer of the present invention can be prepared by any appropriate synthesis method. An example of the synthesis method for a block copolymer in a preferred embodiment of the present invention is as follows. In other words, the block copolymer can be prepared by: forming a polyethylene glycol chain by anion living polymerization using an initiator capable of providing $R^1$; introducing an amino group at the growing end side of the polyethylene glycol chain; polymerizing from the amino end a protected amino acid derivative such as NCA-Lys (TFA) to form a polyamino acid chain segment; deprotecting the side chain of the polyamino acid to expose the amino group; and subjecting the exposed amino group to a reaction with a carboxyl group of a fluorinated carboxyphenylboronic acid to introduce a desired number of FPBA groups into the side chain via an amide bond.

The block copolymer in another preferred embodiment of the present invention may be prepared as follows, for example. In other words, the block copolymer can be prepared by: forming a polyethylene glycol chain by anion living polymerization using an initiator capable of providing $R^1$; introducing an amino group at the growing end side of the polyethylene glycol chain; polymerizing from the amino end an N-carboxylic anhydride of a protected amino acid such as β-benzyl-L-aspartate or γ-benzyl-L-glutamate to form a polyamino acid chain segment; subsequently subjecting the polyamino acid to a reaction with an amine compound such as diethylenetriamine (DET) to introduce an amine residue such as a DET group into the amino acid side chain by an ester-amide exchange reaction; and subsequently subjecting an amino group of the amine residue to a reaction with a carboxyl group of a fluorinated carboxyphenylboronic acid to introduce a desired number of FPBA groups into the side chain via an amide bond. In this process, when the polyamino acid chain segment is formed using β-benzyl-L-aspartate and γ-benzyl-L-glutamate in combination, the subsequent ester-amide exchange reaction occurs preferentially for β-benzyl-L-aspartate. As a result, a block copolymer including an amino acid residue originating from γ-benzyl-L-glutamate as the hydrophobic amino acid residue may be obtained.

It should be noted that part of amino acid ester residues may undergo a structural change by nucleophilic attack of an amine (for example, imide ring formation through the dealcoholization of an amino acid ester residue) during the synthesis process. In the present invention, the polyamino acid chain segment may further include residues that have undergone such structural change. In this case, the residues that have undergone the structural change are excluded from the cationic amino acid residue, the substituted PBA group-containing amino acid residue, and the hydrophobic amino acid residue. Further, part of NH groups and $NH_2$ groups in the cationic amino acid residue may be converted into a salt (mainly a hydrochloride) by use of an acid (mainly hydrochloric acid) during the synthesis process. In the present invention, the polyamino acid chain segment may include such structure. In other words, part of NH groups and $NH_2$ groups in the groups Q, $R^{6a}$, and $R^{6b}$ may be in a form of a salt (for example, a hydrochloride).

In addition, a block copolymer including a hydrophilic polymer (polyethylene glycol) having a target binding site at the end can be synthesized by: synthesizing a block copolymer as mentioned above using polyethylene glycol having a target binding site at the α-end or synthesizing a block copolymer as mentioned above using polyethylene glycol having a functional group capable of subsequently introducing a group including a target binding site into the α-end; and then introducing the group including the target binding site. A variety of methods may be used as introduction methods for the group including the target binding site. For example, when a block copolymer having a polyethylene glycol chain acetalized at the α-end and a compound having a desired target binding site and a cysteine terminus are mixed in an acidic solution, the target binding site can be given at the terminus of the polyethylene glycol chain side.

[B. Complex]

The complex of the present invention is a complex of the block copolymer described in the section A above and a nucleic acid. The complex may be a PIC of a plurality of the block copolymers and the nucleic acids. In addition, the PIC may have a form of a polymer micelle in which the nucleic acids are encapsulated in the plurality of the block copolymers. A related-art carrier has a problem in that the complex dissociates in a medium having an ion concentration almost the same as that of a physiological condition. However, the complex of the present invention is excellent in stability, and hence can maintain the form of the complex in the medium, and even in a medium containing a protein.

As mentioned above, the block copolymer has a cationic group in a side chain of the amino acid in the polyamino acid chain segment, and hence the block copolymer can form a stable complex (for example, vesicle or associate) with

a small molecular weight nucleic acid as well under a physiological condition. The nucleic acid capable of forming a complex with the block copolymer means a poly- or oligonucleotide including nucleotides formed of a purine or pyrimidine base, a pentose, and phosphoric acid as basic units, and examples thereof may include oligo- or poly-double-stranded RNA, oligo- or poly-double-stranded DNA, oligo- or poly-single-stranded DNA, and oligo- or poly-single-stranded RNA. Further, oligo- or poly-double-stranded nucleic acid and oligo- or poly-single-stranded nucleic acid in each of which RNA and DNA exist in a mixed state in the same chain are also included. The nucleotide contained in the nucleic acid may be of a natural type or of a chemically modified non-natural type, or may have added thereto an amino group, a thiol group, a fluorescent compound, or any other molecule.

When the nucleic acid is RNA, the RNA includes 3' -terminal ribose having a 1,2-cis-diol, and hence can be reversibly bound covalently to the block copolymer via the substituted PBA group as well as the electrostatic interaction with the cationic group of the cationic amino acid residue. As a result, a complex excellent in stability is obtained. Such complex excellent in stability enables prevention of replacement of RNA molecules with a blood level (usually, about 4 to 7 mM) of glucose (having a cis-diol structure in its molecule), and hence is excellent in stability in blood. Therefore, when the complex is administered to a living body, the retentivity in blood may be improved. In particular, when the nucleic acid is double-stranded RNA, a cross-linked structure having as a binding site 3'-terminal ribose of each chain may be formed in the micelle. Therefore, a complex that is very excellent instabilitycanbeobtained. Inaddition, when the complex including the RNA is taken up into an endosome that is under a low-pH environment, the substituted PBA group is hydrophobized to increase a hydrophobic interaction, resulting in further improved stability. Further, after transfer from the endosome to a cytoplasm, the RNA can be released rapidly by replacing another molecule that is present in the cytoplasm at a relatively high concentration and has a 1,2-cis-diol, such as ATP or ribonucleic acid.

The strand length of the nucleic acid maybe, for example, 4 to 20,000 bases, preferably 10 to 10,000 bases, more preferably 18 to 30 bases.

In consideration of functions or actions, examples of the nucleic acid may include plasmid DNA, siRNA, micro RNA, shRNA, an antisense nucleic acid, a decoy nucleic acid, an aptamer, and a ribozyme.

The ratio of the content of the block copolymer to the content of the nucleic acid in the complex of the present invention may be appropriately set depending on the kinds of the block copolymer and the nucleic acid, the application of the complex, or the like. When the nucleic acid is siRNA, the complex of the present invention has anN/P ratio of preferably 2 or more from the viewpoint of improving stability under a physiological condition, or has an N/P ratio of preferably 200 or less from the viewpoint of suppressing toxicity due to the polymer. It should be noted that the N/P ratio means [concentration of amino group of polyamino acid side chain in block copolymer contained in complex] / [concentration of phosphate group in nucleic acid]. Further, from the viewpoint of improving stability under a physiological condition, when the nucleic acid is siRNA, the complex of the present invention may have [the number of positive charges in the polyamino acid chain segment] / [the total of the number of negative charges in the polyamino acid chain segment and the number of negative charges in siRNA] at pH 7.4 of, for example, 1/2 to 20/1, preferably 1/1 to 10/1.

The block copolymer may maintain high hydrophilicity by appropriately selecting the kinds of substituted PBA groups and the number of the groups introduced, and hence the complex of the present invention can be prepared, for example, only by mixing the block copolymer and the nucleic acid in an aqueous solution buffered as necessary. In other words, the complex of the present invention may have advantages in that it is not necessary to further modify the terminus of the block copolymer and in that the complex can be prepared by a simple method.

## Examples

Hereinafter, the present invention is specifically described by way of Examples. However, the present invention is not limited to Examples below. It should be noted that in Examples below, polymer structures are described in the order of "the molecular weight (kDa) of PEG-the number of the FPBA group-containing amino acid residues/the total number of the amino acid residues constituting the polyamino acid chain segment". For example, when PEG has a molecular weight of 10,000, and the polyamino acid chain segment is a polyamino acid chain segment obtained by introducing 5 FPBA groups into polylysine having a polymerization degree of 40, the polymer is represented as "PEG-PLL 10-5/40." Further, for example, when PEG has a molecular weight of 10, 000, and the polyamino acid chain segment is polylysine that has no FPBA group introduced and has a polymerization degree of 40, the polymer is represented as "PEG-PLL 10-0/40". In this context, the numbers represented are average values in the whole of the block copolymers.

[Preparation of polylysine-based block copolymer]

A synthesis scheme A for a polylysine-based block copolymer used in the following experimental example is shown below. Specifically, the block copolymer was prepared as mentioned in (1-i) to (1-iii).

[Chem. 9]

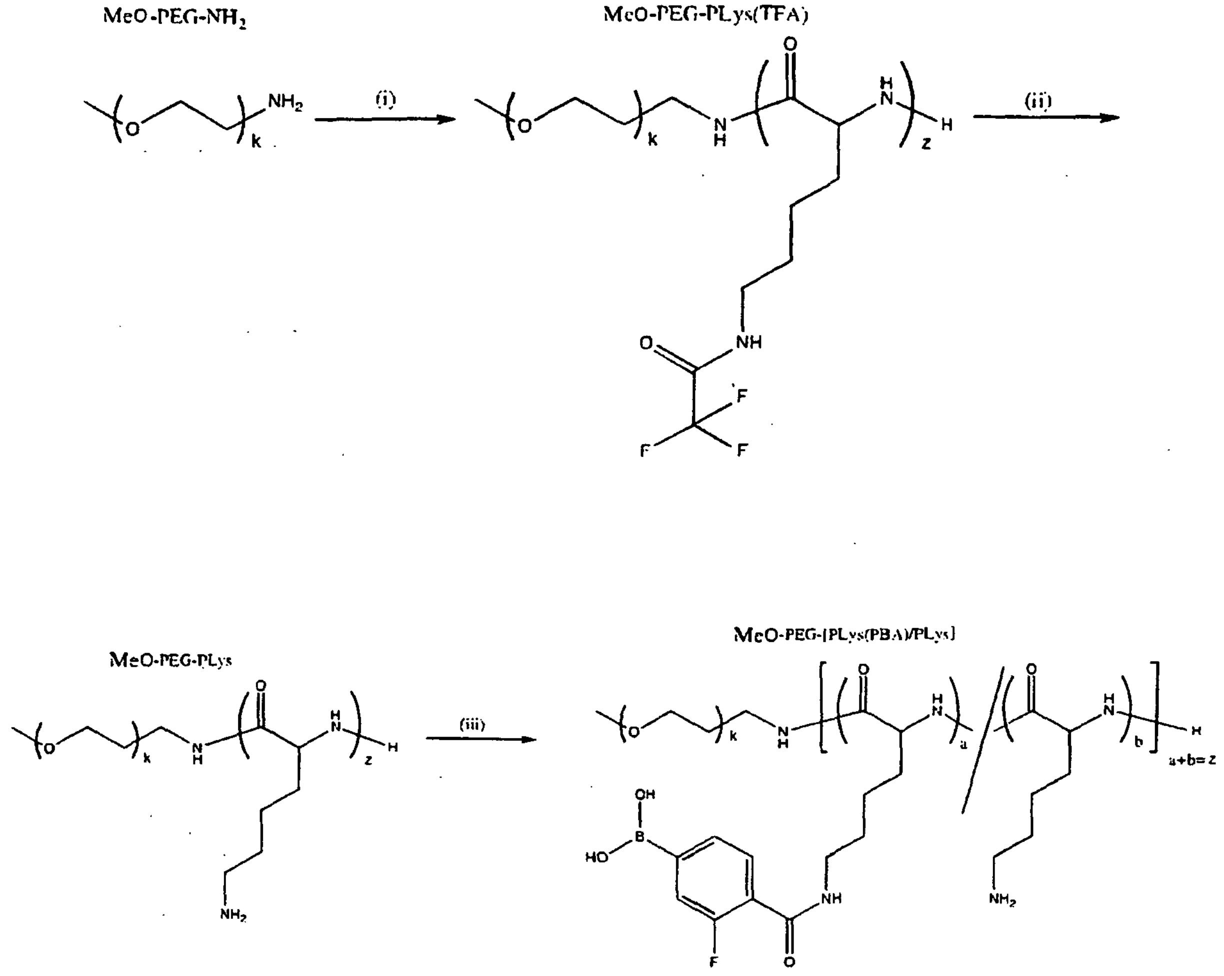


Synthesis scheme A

(1-i) Methoxy-PEG-$NH_2$ is lyophilized from benzene and dissolved in dimethylformamide (DMF) containing 1 M thiourea. To the resultant solution is added NCA-Lys(TFA) dissolved in DMF containing 1 M thiourea, and the mixture is stirred for 3 days at 25°C to perform polymerization. After the polymerization, the reaction solution is added dropwise to diethyl ether, and the resultant precipitates are collected by filtration and dried under reduced pressure to afford MeO-PEG-PLys(TFA).

(1-ii) MeO-PEG-PLys(TFA) is stirred in 1 N NaOHaq./methanol=1/10 at 35°C for 12 hours, and the solution is subjected to dialysis (external solution: water) and lyophilized to afford MeO-PEG-PLys.

(1-iii) MeO-PEG-PLys and D-Mannitol in an amount of 5 equivalents relative to amino groups of PLys are dissolved in a 50 mM $NaHCO_3$ aqueous solution. To the resultant solution is added 3-fluoro-4-carboxyphenylboronic acid dissolved in methanol, and a condensing agent DMT-MM is added thereto. The resultant solution is stirred at room temperature the whole night, subj ected to dialysis (external solution: water), and lyophilized to afford MeO-PEG-[PLys(FPBA)/PLys].

The molecular weight of the resultant block copolymer was measured by gel permeation chromatography (GPC). In this case, a solution obtained by dissolving D-sorbitol in 50 mM $NaHCO_3$ at 50 mg/mL or more, a phosphate buffer (pH 5 to 7), or a mixed solvent of a 500 mM NaCl aqueous solution containing 50 mg/mL D-sorbitol and methanol

(NaCl aq./methanol=1/4) was used as a mobile phase. Further, the amount of the FPBA group introduced was estimated from an integration ratio of the side chain to the phenyl ring by $^1$H-NMR spectra. In this case, a solution obtained by dissolving the polymer in $D_2O$ containing a small amount of D- sorbitol was used as a measurement sample.

[Preparation of PAsp(DET)-based block copolymer]

A synthesis scheme B for a PAsp(DET)-based block copolymer used in the following experimental example is shown below. Specifically, the block copolymer was prepared as mentioned in (2-i) to (2-iii).

[Chem. 10]

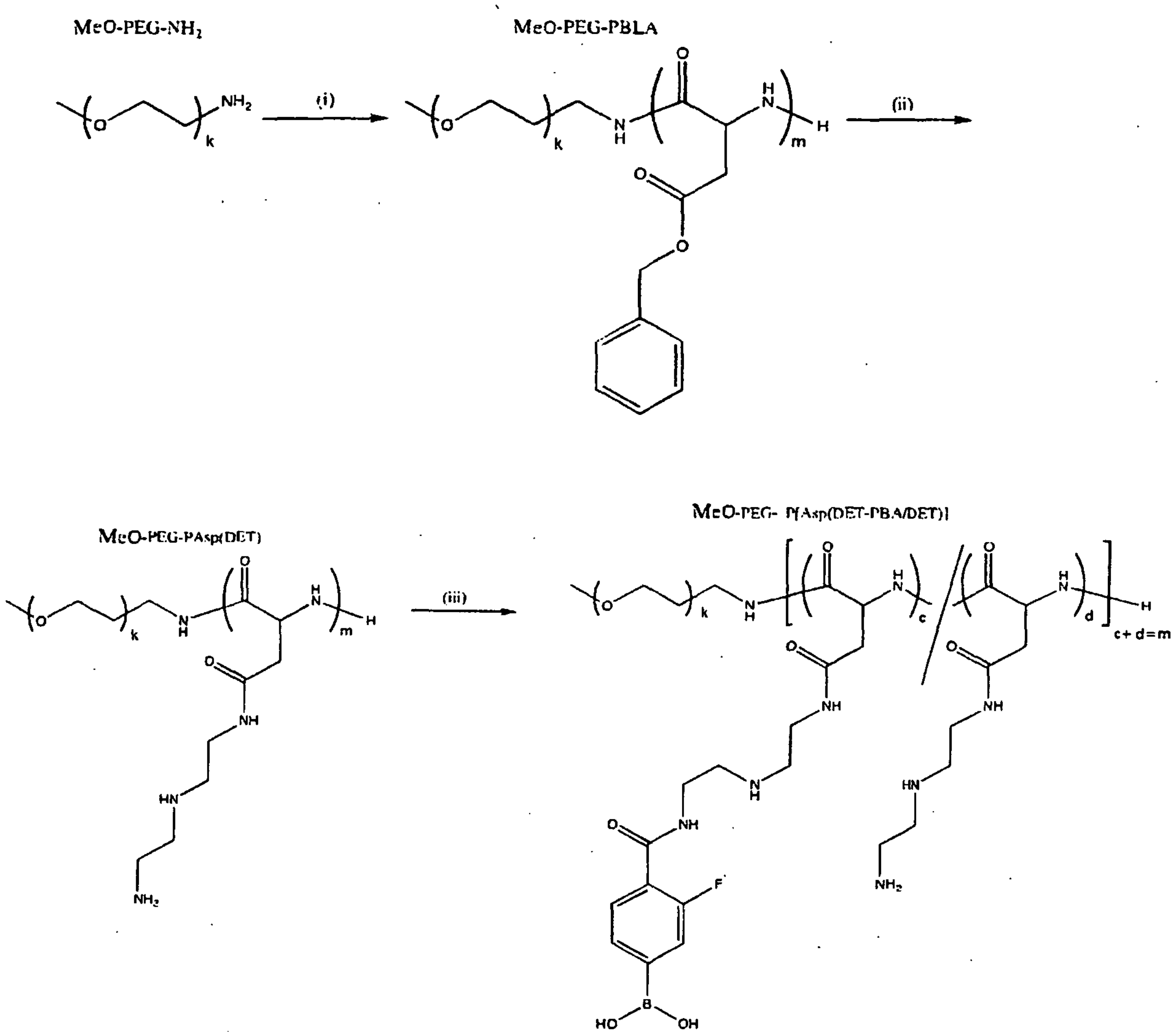


Synthesis scheme B

(2-i) Methoxy-PEG-$NH_2$ is lyophilized from benzene and dissolved in dichloromethane. NCA-BLA dissolved in DMF/dichloromethane=1/10 is added to the resultant solution, and the mixture is stirred for 3 days at 35°C to perform polymerization. After the polymerization, the reaction solution is added dropwise to diethyl ether, and the resultant precipitates are collected by filtration and dried under reduced pressure to afford MeO-PEG-PBLA.

(2-ii) MeO-PEG-PBLA is lyophilized from benzene and dissolved in N-methyl-2-pyrrolidone (NMP). The resultant solution is added dropwise to a solution of diethylenetriamine in NMP, and the mixture is stirred at 5 to 10°C for 1 hour. The resultant mixture is neutralized with hydrochloric acid under ice cooling, subjected to dialysis (external solution: 0.01 N hydrochloric acid), and lyophilized to afford MeO-PEG-PAsp(DET).

(2-iii) MeO-PEG-PAsp(DET) and D-Mannitol in an amount of 5 equivalents relative to primary amines of PAsp(DET) are dissolved in a 50 mM $NaHCO_3$ aqueous solution under ice cooling (0°C). To the resultant solution is added 3-fluoro-4-carboxyphenylboronic acid dissolved inmethanol, and a condensing agent DMT-MM is added thereto. The resultant solution is stirred for 6 hours, subjected to dialysis at 5°C (external solution: 0.01 N hydrochloric acid optionally containing sorbitol), and lyophilized to afford MeO-PEG-P[Asp(DET-FPBA/DET)].

The molecular weight of the resultant block copolymer was measured by gel permeation chromatography (GPC). In this case, a solution obtained by dissolving D-sorbitol in a 50 mM phosphate buffer (pH 7.4) at 50 mg/mL was used as a mobile phase. Further, the amount of the FPBA group introduced was estimated from an integration ratio of the side chain to the phenyl ring by $^1$H-NMR spectra. In this case, a solution obtained by dissolving the polymer in $D_2O$ containing a small amount of NaOD was used as a measurement sample.

[siRNA]

Sequences of siRNAs used in the following experimental examples are as follows. Labels such as Cy3 were all introduced into the 5' ends of sense strands.

(1) hVEGF-siRNA (siRNA for human vascular endothelial growth factor):

Sense strand: 5'-GAUCUCAUCAGGGUACUCCdTdT-3' (SEQ ID NO: 1)
Antisense strand: 5'-GGAGUACCCUGAUGAGAUCdTdT-3' (SEQ ID NO: 2)

(2) Scramble-siRNA (siRNA having a sequence for nontherapeutic use) :

Sense strand: 5'-UUCUCCGAACGUGUCACGUUU-3' (SEQ ID NO: 3)
Antisense strand: 5'-ACGUGACACGUUCGGAGAAUU-3' (SEQ ID NO: 4)

(3) GL3-siRNA (siRNA for firefly luciferase)

Sense strand: 5'-CUUACGCUGACUACUUCGAUU-3' (SEQ ID NO: 5)
Antisense strand: 5'-UCGAAGUACUCAGCGUAAGW-3' (SEQ ID NO: 6)

[Relationship between FPBA group content and complex stability]

A variety of block copolymers having different amino acid polymerization degrees and FPBA group contents were prepared as mentioned in the sections (1-i) to (1-iii) and (2-i) to (2-iii). It should be noted that PEG having a molecular weight (Mw) of 12, 000 was used as methoxy-PEG-$NH_2$. The block copolymers were mixed with siRNA fluorescently labeled with Cy3 so as to achieve an N/P ratio of 8. The resultant mixtures were left to stand still in a refrigerator for 1 to 2 hours and diluted with a serum solution (150 mM NaCl, 10 mM Hepes, 10% FBS) so as to achieve an siRNA concentration of 50 nM. The resultant diluted solutions were left to stand still at room temperature for about 1 hour, and the siRNA diffusion time was measured with a confocal laser scanning microscope (manufacturedby Carl Zeiss, product name "LSM510") (10 sec×10 times). Table 1 and FIG. 1 show the results. It should be noted that when measurement was carried out in the same manner as above except that the siRNA was not mixed with the block copolymer, the diffusion time (diffusion time of siRNA alone) was 157.5 μsec. It should be noted that GL3-siRNA was used as the siRNA.

[Table 1]

| PLys-based block copolymer | | | | | | | | | | |
|---|---|---|---|---|---|---|---|---|---|---|
| Molecular weight of PEG chain | 12,000 | | | | | | | | | |
| Total number of amino acid residues in polyamino acid chain segment | 21 | | 43 | | | | | 54 | | |
| Number of cationic amino acid residues free of FPBA group in polyamino acid chain segment | 18.0 | 16.3 | 43.0 | 35.3 | 32.9 | 28.8 | 24.1 | 51.1 | 43.3 | 38.1 |
| Number of FPBA groups in polyamino acid chain segment | 3.0 | 4.7 | 0.0 | 7.7 | 10.1 | 14.2 | 18.9 | 2.9 | 10.7 | 15.9 |
| Diffusion time [µsec, N/P=8] | 323.3 | 331.4 | 298.6 | 321.1 | 440.2 | 773.9 4 | 783.6 | 337.0 | 434.9 | 1,002 .2 |
| √ (Number of cationic amino acid residues free of FPBA group) + 2√ (Number of FPBA groups) | 7.7 | 8.4 | 6.6 | 11.5 | 12.1 | 12.9 | 13.6 | 10.6 | 13.1 | 14.1 |
| PLys-based block copolymer | | | | | | | | | | |
| Molecular weight of PEG chain | 12,000 | | | | | | | | | |
| Total number of amino acid residues in polyamino acid chain segment | 74 | | | | | | 106 | | | |
| Number of cationic amino acid residues free of FPBA group in polyamino acid chain segment | 74.0 | 70.7 | 68.2 | 65.9 | 62.1 | 56.9 | 106.0 | 104.5 | 101.9 | 93.7 |
| Number of FPBA groups in polyamino acid chain segment | 0.0 | 3.3 | 5.8 | 8.1 | 11.9 | 17.1 | 0.0 | 1.5 | 4.1 | 12.3 |
| Diffusion time [µsec, N/P=8] | 379.1 | 1,326.2 | 553.1 | 764.2 | 1,073.0 | 1,238.9 | 382.4 | 2,120 .6 | 1,778 .7 | 2,896.3 |
| √(Number of cationic amino acid residues free of FPBA group) +2√ (Number of FPBA groups) | 8.6 | 12.1 | 13.1 | 13.8 | 14.8 | 15.8 | 10.3 | 12.7 | 14.2 | 16.7 |
| PAsp(DET)-based block copolymer | | | | | | | | | | |
| Molecular weight of PEG chain | 12,000 | | | | | | | | | |
| Total number of amino acid residues in polyamino acid chain segment | 35.6 | 33.9 | 34.5 | 57.4 | 74.1 | 74.0 | | | | |
| Number of cationic amino acid residues free of FPBA group in polyamino acid chain segment | 32.9 | 23.7 | 14.5 | 48.1 | 69.9 | 67.0 | | | | |
| Number of FPBA groups in polyamino acid chain segment | 2.6 | 10.2 | 20.0 | 9.3 | 4.2 | 7.0 | | | | |
| Diffusion time [µsec, N/P=8] | 313.3 | 367.6 | 807.8 | 1,241.9 | 1,622.6 | 1,41. 7 | | | | |

(continued)

| PAsp(DET)-based block copolymer | | | | | | | |
|---|---|---|---|---|---|---|---|
| √(Number of cationic amino acid residues free of FPBA group) +2√ (Number of FPBA groups) | 9.0 | 11.3 | 12.7 | 13.0 | 12.4 | 13.5 | |
| In the above Table 1, the block copolymers having 0.0 FPBA group in polyamino acid chain segment represent comparative examples. | | | | | | | |

As shown in Table 1 and FIG. 1, when siRNA is mixed with the block copolymers, the siRNA diffusion time was longer than that when siRNA was used singly. Therefore, all of the block copolymers were found to form complexes with siRNA. In addition, the diffusion time with the block copolymers having the FPBA group introduced was longer than that with the block copolymers free of the FPBA group, and hence the block copolymers having the FPBA group introduced were found to form more stable complexes. Of those, when the block copolymers satisfying the relationship of [√ (the number of cationic amino acid residues free of the FPBA group in the polyamino acid chain segment) +2√ (the number of FPBA groups in the polyamino acid chain segment) $\geq$12.0] were used, the siRNA diffusion time was 400 $\mu$sec or more, suggesting that highly stable complexes were formed.

[Toxicity test of block copolymer]

A549-Luc cells were seeded in a 96-well culture dish at 2, 500 cells/well and cultured in a DMEM medium containing 10% fetal bovine serum in an incubator for 24 hours. The medium was exchanged for a fresh DMEM medium containing 10% fetal bovine serum, and each of the block copolymers dissolved in physiological saline was added at different amine concentrations. Subsequently, the cells were cultured for 48 hours, the number of living cells were measured with "Cell Counting Kit 8" (product name, manufactured by Dojindo Molecular Technologies, Inc.), and a cell survival rate thereof was calculated (N=4). FIG. 2 shows the results.

[Toxicity test of complex]

A549-Luc cells were seeded in a 96-well culture dish at 2,500 cells/well and cultured in a DMEM medium containing 10% fetal bovine serum in an incubator for 24 hours. The medium was exchanged for a fresh DMEM medium containing 10% fetal bovine serum, and each of the complexes of siRNA and the block copolymers was added at different siRNA concentrations. Subsequently, the cells were cultured for 48 hours, the number of living cells were measured with "Cell Counting Kit 8" (product name, manufactured by Dojindo Molecular Technologies, Inc.), and a cell survival rate thereof was calculated (N=4). FIG. 3 shows the results. It should be noted that the complexes were prepared by: adding the block copolymers and GL3-siRNA to a 10 mM HEPES buffer (pH 7.3) so as to achieve an N/P ratio of 4 and an siRNA concentration of 80 nM; mixing the resultant; leaving the mixture to stand still for about 1 to 2 hours at room temperature; and diluting the mixture to different concentrations.

As shown in FIG. 2, the groups treated with the block copolymers having the FPBA group introduced had higher cell survival rates as compared to the group treated with the block copolymer having no FPBA group introduced. In addition, as shown in FIG. 3, the groups treated with the complexes including the block copolymers having the FPBA group introduced had higher cell survival rates as compared to the group treated with the complex including the block copolymer having no FPBA group introduced. The results reveal that the introduction of the FPBA group causes no cytotoxicity, and the block copolymers having the FPBA group introduced have satisfactory biocompatibility.

[Evaluation of pH stability of complex]

A block copolymer (PEG-PLL 12-23/43) and GL3-siRNA labeled with Cy3 were dissolved in a buffer (phosphate buffer or phosphate-citrate buffer) having a variety of pH values, and mixed so as to achieve an N/P ratio of 4 and an siRNA concentration of 50 nM. The resultant mixtures were left to stand still for about 1 hour at room temperature, and the siRNA diffusion time was measured with the confocal laser scanning microscope (manufactured by Carl Zeiss, product name "LSM510") (10 sec$\times$10 times). FIG. 4 shows the results.

As shown in FIG. 4, the siRNA diffusion time was longer than 800 $\mu$sec in a wide pH range of from a body environment (about pH 7.4) to an acidic environment (about pH 5.5) of the inside of an endosome or the like. Therefore, the block copolymer of the present invention was found to be able to form a stable complex with siRNA (siRNA-encapsulated polymer micelle) in the pH range.

[Evaluation of siRNA-releasing property of complex]

The block copolymer (PEG-PLL 12-23/43) and GL3-siRNA labeled with Cy3 were separately dissolved in a 10 mM HEPES buffer (pH 7.3), and mixed so as to achieve an N/P ratio of 4. The resultant mixtures were left to stand still for about 1 to 2 hours in a refrigerator, and diluted with 10 mM HEPES buffers (pH 7.3) containing a variety of concentrations of glucose, dTMP, or UMP so as to achieve an siRNA concentration of 50 nM. The resultant diluted solutions were left to stand still at room temperature for about 1 hour. Subsequently, the siRNAdif fusion time was measured with the confocal laser scanning microscope (manufactured by Carl Zeiss, product name "LSM510") (10 sec$\times$10 times). FIG. 5 shows the results.

As shown in FIG. 5, the siRNA diffusion time at glucose concentrations almost the same as those in blood

(usually, about 4 to 7 mM) is longer than 800 μsec, which shows that the block copolymer of the present invention can form a stable complex with siRNA (siRNA-encapsulated polymer micelle) in blood. In addition, when the glucose concentrations are higher than that in blood, the diffusion time gradually becomes shorter, suggesting that the micelles collapse at such concentrations to rapidly release siRNA. It should be noted that such collapse of the micelles is estimated to occur by replacement of RNA molecules bound to the FPBA groups via the 1,2-diol structures in the micelles with glucose having the 1,2-diol structure like the RNA molecules.

Similarly, it is found that the micelle shape can be maintained in the presence of UMP having the 1,2-diol structure at low UMP concentrations, but the micelles collapse at UMP concentrations higher than 1 mM by replacement of the RNA molecules with UMP to rapidly release siRNA. On the other hand, the micelle shape can be maintained stably in the presence of dTMP having a structure similar to UMP but having no 1,2-diol structure, even at dTMP concentrations higher than 10 mM. This is probably because no replacement of the RNA molecules occurs.

[Evaluation of intracellular uptake ability of complex]

A549-Luc cells were seeded in a 48-well dish at 10,000 cells/well, and cultured for 24 hours in an incubator with a DMEM medium containing 10% fetal bovine serum. The medium was exchanged for a fresh DMEM medium containing 10% fetal bovine serum, and siRNA labeled with Cy3 or a complex of the siRNA and a block copolymer was added to the medium so that the concentration of siRNA was 100 nM/well. After having been cultured in an incubator at 37°C for 5 hours or 9 hours, the cells were washed three times with 1 mL of a PBS buffer, and detached from the dish with a trypsin-EDTA solution. The detached cells were subjected to histogram analysis using a flow cytometer (manufactured by BD, LSRII) in which a Cy3 filter was set. Thus, the amount of siRNA taken up into the cells was evaluated (N=4). It should be noted that GL3-siRNA was used as siRNA. FIG. 6 shows a graph showing the amount of siRNA taken up into the cells. It should be noted that the complex was prepared by adding each block copolymer and siRNA to a 10 mM HEPES buffer (pH 7.3) so as to achieve an N/P ratio of 8 and an siRNA concentration of 4 μM, mixing the resultant, and leaving the mixture to stand still for about 1 to 2 hours at room temperature.

As shown in FIG. 6, the amounts of siRNA taken up in the case where siRNA was added as a complex with the block copolymer having the FPBA group introduced were dramatically improved as compared to those in the case where siRNA was added singly and in the case where siRNA was added as a complex with the block copolymer having no FPBA group introduced.

[Evaluation of blood retentivity of complex]

The block copolymer (PEG-PLL 12-23/43) and Cy5-GL3-siRNA were added to a 10 mM HEPES buffer (pH 7.3) so as to achieve an N/P ratio of 8 and an siRNA concentration of 7.5 μM, and the resultant was mixed and left to stand still for about 1 to 2 hours at room temperature to prepare a complex solution. The complex solution or a solution of the siRNA (10 mM HEPES buffer) was administered to the tail veins of mice (female Balb/Cnude, 6-week-old, N=4) so that the dose was 20 μg of the siRNA, and the amount of the siRNA remaining in blood was determined over time. FIG. 7 shows the results.

As shown in FIG. 7, when siRNA was administered singly, only about 10% of siRNA remained at 10 minutes after administration, and almost no siRNA remained after a lapse of 1 hour. On the other hand, when siRNA was administered as the complex, 40% or more of siRNA remained at 1 hour after administration, and 10% or more of siRNA remained even after a lapse of 2 hours. This suggests that the complex including the block copolymer of the present invention and siRNA (siRNA-encapsulated micelle) is very excellent in retentivity in blood.

[Evaluation of anticancer activity of complex]

(1) Preparation of FPBA group-containing block copolymer

A block copolymer Ace-PEG-PAsp (DET) 12-62/75 was obtained by a scheme similar to the scheme B. Specifically, Ace-PEG-PBLA (12-110) having an acetal group at the terminus on the PEG side was subjected to aminolysis with DET to afford Ace-PEG-PAsp(DET) (12-90). Subsequently, dialysis was carried out against an NaCl solution to convert acetate serving as a counterion into chloride, and 4-carboxy-3-fluorophenylboronic acid was condensed using a condensing agent (DMT-MM) to afford a block copolymer Ace-PEG-PAsp(DET) 12-62/75.

(2) Preparation of cRGD-introduced block copolymer

Subsequently, cRGD peptide (Cyclic (Arg-Gly-Asp-D-Phe-Cys)) was mixed with DTT in an amount of 5 equivalents relative to the acetal group at pH 7.2 for 1 hour, and the resultant mixture and the block copolymer were mixed,

and the pH was adjusted to 2. The mixture was stirred for 1 hour, and the pH was adjusted to 5 with 2 M sodium acetate and NaOH. After that, water was added thereto so as to achieve a polymer concentration of 20 mg/mL, and the mixture was stirred overnight. Dialysis was carried out against pure water to remove unreacted products and unwanted matters, followed by lyophilization to afford a block copolymer cRGD-PEG-PAsp(DET) 12-62/75 having cRGD introduced at the terminus of the PEG chain. It should be noted that the reaction and the dialysis were carried out at 4°C (refrigerator).

(3) Preparation of complex

Block copolymers were dissolved in a 10 mM HEPES buffer so as to achieve a concentration of 10 mg/mL. 12.18 μL of the polymer solutions, 75 μL of siRNA (15 μM in a 10 mM HEPES buffer), and 7.81 μL of a 10 mM HEPES buffer were mixed, and the mixtures were left to stand still overnight. Several hours prior to administration, 5 μL of 3 M NaCl in a 10 mM HEPES buffer were added to the mixtures, and the concentration of NaCl was adjusted to 150 mM (total volume: 100 μL). Thus, complexes (siRNA-encapsulated polymer micelles) were obtained. Table 2 shows combinations of siRNA and the block copolymers and mixing ratios in the complexes prepared.

[Table 2]

| Complex | Block copolymer | siRNA | Mixing ratio*1 | Average diameter |
|---|---|---|---|---|
| (+)hVEGF | cRGD-PEG-PAsp(DET) 12-62/75 | siRNA-hVEGF | 5 | - |
| (-)hVEGF | Ace-PEG-PAsp(DET) 12-62/75 | siRNA-hVEGF | 5 | 39.95 nm |
| (+)Scramble | cRGD-PEG-PAsp(DET) 12-62/75 | siRNA-Scramble | 5 | - |
| *1: Ratio of number of positive charges of polyamino acid chain segment to total of number of negative charges of polyamino acid chain segment and number of negative charges of siRNA (positive charges/total of negative charges) at pH 7.4 | | | | |

(4) Evaluation of anticancer activity

6-week-old mice (female Balb/C nude, N=4) were purchased and fed for 1 week, and Hela-luc cells adjusted to 5. $0\times10^7$ cells/mL were injected subcutaneously in an amount of 100 μL per mouse. After that, the mice were further fed for 4 days, and treatment (i.e., administration of a complex) was started. Specifically, the complex prepared in (3) above was administered to the tail veins of the mice every other day until day 6 (i.e., when the administration start date was defined as day 0, the complex was administered four times in total on day 0, day 2, day 4, and day 6). The siRNA was administered in a dose of 15 μg/100 μL. To a control group, a HEPES solution was administered in a dose of 100 μL. FIG. 8 shows a relationship between a relative value of a tumor volume to a tumor volume on the administration start date and the number of days elapsed after administration.

As shown in FIG. 8, in the group to which the (-)hVEGF complex using as a carrier the block copolymer of the present invention in which the PEG terminus had no cRGD introduced was administered, growth of the tumor was clearly suppressed as compared to the control group and the group to which the (+) Scramble complex was administered. This reveals that siRNA was maintained stably in blood by the block copolymer and exhibited RNA interference ability. Further, the group to which the (+)hVEGF complex using as a carrier the block copolymer in which the PEG terminus had cRGD introduced was administered, growth of the tumor was significantly suppressed as compared to the control group and the group to which the (+) Scramble complex was administered. This reveals that the complex is excellent in stability in blood, and is taken up into tumor tissues with a high transfer rate to efficiently exhibit the RNA interference effect.

Industrial Applicability

The block copolymer of the present invention can be suitably applied in the DDS field.

## Claims

**1.** A pharmaceutical composition, comprising a complex of:

a block copolymer including a polyamino acid chain segment and a hydrophilic polymer chain segment; and
a nucleic acid,
wherein:

the polyamino acid chain segment includes an amino acid residue having a cationic group in a side chain and an amino acid residue having a substituted phenylboronic acid group in a side chain, the substituted phenylboronic acid group having a phenyl ring in which at least one hydrogen atom is substituted with a halogen or a nitro group;
the substituted phenylboronic acid group is introduced into the side chain of the amino acid residue via an amide bond, a carbamoyl bond, an alkyl bond, an ether bond, an ester bond, a thioester bond, a thioether bond, a sulfonamide bond, a urethane bond, a sulfonyl bond, a thymine bond, a urea bond, or a thiourea bond;
the substituted phenylboronic acid group has a pKa of less than 7.5; and
the nucleic acid forms a reversible covalent bond with the substituted phenylboronic acid group.

**2.** A pharmaceutical composition according to claim 1, wherein the substituted phenylboronic acid group comprises a fluorinated phenylboronic acid group represented by the following formula (I):

[Chem. 1]

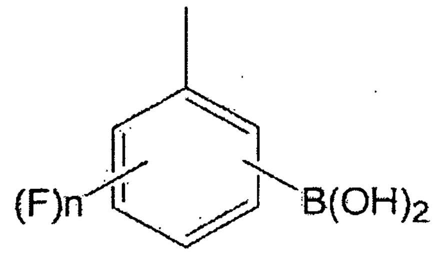

(I)

in the formula (I): F's are present independently; n represents 1, 2, 3, or 4; and when n represents 1, F and $B(OH)_2$ may be introduced at any one of ortho, meta, and para positions.

3. A pharmaceutical composition according to claim 1 or 2, wherein the cationic group comprises an amino group.

4. A pharmaceutical composition according to any one of claims 1 to 3, wherein the amino acid residue having a cationic group in a side chain is a lysine residue or an amino acid residue obtained by substituting an -OH moiety in a carboxyl group (-C(=O)OH) of an acidic amino acid by any one of groups of the following formulas (i) to (iv):

- $NH-(CH_2)_{p1}-[NH-(CH_2)_{q1}-]_{r1}NH_2$ (i);
- $NH-(CH_2)_{p2}-N[-(CH_2)_{q2}-NH_2]_2$ (ii);
- $NH-(CH_2)_{p3}-N\{[-(CH_2)_{q3}-NH_2][-(CH_2)_{q4}-NH-]_{r2}H\}$ (iii); and
- $NH-(CH_2)_{p4}-N\{-(CH_2)_{q5}-N[-(CH_2)_{q6}-NH_2]_2\}_2$ (iv)

in the formulas (i) to (iv): p1 to p4, q1 to q6, and r1 and r2 each independently represent an integer of from 1 to 5.

5. A pharmaceutical composition according to any one of claims 1 to 4, wherein a number of amino acid residues that have a cationic group and are free of the substituted phenylboronic acid group and a number of the substituted phenylboronic acid groups in the polyamino acid chain segment satisfy the following relationship.

[Math. 1]

$$\sqrt{\text{Number of cationic amino acid residues free of substituted phenylboronic acid group in polyamino acid chain segment}} + 2\sqrt{\text{Number of substituted phenylboronic acid groups in polyamino acid chain segment}} \geqq 12.0$$

6. A pharmaceutical composition according to any one of claims 1 to 5, wherein the polyamino acid chain segment further includes an amino acid residue having a hydrophobic group in a side chain.

7. A block copolymer comprising:

a polyamino acid chain segment; and
a hydrophilic polymer chain segment,
wherein:

the polyamino acid chain segment includes an amino acid residue having a cationic group in a side chain and an amino acid residue having a substituted phenylboronic acid group in a side chain, the substituted phenylboronic acid group having at least one hydrogen atom of a phenyl ring substituted with a halogen or a nitro group;
the substituted phenylboronic acid group is introduced into the side chain of the amino acid residue via an amide bond, a carbamoyl bond, an alkyl bond, an ether bond, an ester bond, a thioester bond, a thioether bond, a sulfonamide bond, a urethane bond, a sulfonyl bond, a thymine bond, a urea bond, or a thiourea bond;
the substituted phenylboronic acid group has a pKa of less than 7.5; and
a number of amino acid residues that have a cationic group and are free of the substituted phenylboronic

acid group and a number of the substituted phenylboronic acid groups in the polyamino acid chain segment satisfy the following relationship.

[Math. 2]

$$\sqrt{\text{Number of cationic amino acid residues free of substituted phenylboronic acid group in polyamino acid chain segment}} + 2\sqrt{\text{Number of substituted phenylboronic acid groups in polyamino acid chain segment}} \geqq 12.0$$

**Patentansprüche**

1. Pharmazeutische Zusammensetzung, umfassend einen Komplex aus:

    einem Blockcopolymer, enthaltend ein Polyaminosäurekettensegment und ein hydrophiles Polymerkettensegment; und
    einer Nukleinsäure,
    wobei:

        das Polyaminosäurekettensegment einen Aminosäurerest mit einer kationischen Gruppe in einer Seitenkette und einen Aminosäurerest mit einer substituierten Phenylboronsäuregruppe in einer Seitenkette enthält, wobei die substituierte Phenylboronsäuregruppe einen Phenylring aufweist, in dem mindestens ein Wasserstoffatom durch ein Halogen oder eine Nitrogruppe substituiert ist;
        die substituierte Phenylboronsäuregruppe in die Seitenkette des Aminosäurerests durch eine Amidbindung, eine Carbamoylbindung, eine Alkylbindung, eine Etherbindung, eine Esterbindung, eine Thioesterbindung, eine Thioetherbindung, eine Sulfonamidbindung, eine Urethanbindung, eine Sulfonylbindung, eine Thyminbindung, eine Harnstoffbindung oder eine Thioharnstoffbindung eingeführt ist;
        die substituierte Phenylboronsäuregruppe einen pKa von weniger als 7,5 aufweist; und
        die Nukleinsäure eine reversible kovalente Bindung mit der substituierten Phenylboronsäuregruppe bildet.

2. Pharmazeutische Zusammensetzung nach Anspruch 1, wobei die substituierte Phenylboronsäuregruppe eine fluorierte Phenylboronsäuregruppe, dargestellt durch die folgende Formel (I), umfasst:

[Chem. 1]

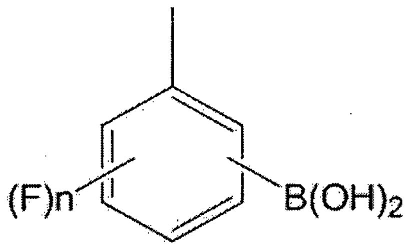

(I)

wobei in der Formel (I): die Fs unabhängig voneinander vorhanden sind; n 1, 2, 3 oder 4 ist; und wenn n 1 ist, können F und $B(OH)_2$ an einer beliebigen Position aus ortho-, meta- und para-Positionen eingeführt sein.

3. Pharmazeutische Zusammensetzung nach Anspruch 1 oder 2, wobei die kationische Gruppe eine Aminogruppe umfasst.

4. Pharmazeutische Zusammensetzung nach einem der Ansprüche 1 bis 3, wobei der Aminosäurerest mit einer kationischen Gruppe in einer Seitenkette ein Lysinrest oder ein Aminosäurerest, erhalten durch Substituieren einer -OH-Gruppe in einer Carboxygruppe (-C(=O)OH) einer sauren Aminosäure durch eine der Gruppen der folgenden

Formeln (i) bis (iv), ist:

- $NH-(CH_2)_{p1}-[NH-(CH_2)_{p1}-]_{r1}NH2$ (i);
- $NH-(CH_2)_{p2}-N[-(CH_2)_{p2}-NH_2]_2$ (ii);
- $NH-(CH_2)_{p3}-N\{[-(CH_2)_{q3}-NH_2][-(CH_2)_{q4}-NH-]_{r2}H\}$ (iii); und
- $NH-(CH_2)_{p4}-N\{-(CH_2)_{q5}-N[-(CH_2)_{q6}-NH_2]_2\}_2$ (iv)

wobei in den Formeln (i) bis (iv): p1 bis p4, q1 bis q6 und r1 und r2 jeweils unabhängig voneinander eine ganze Zahl von 1 bis 5 sind.

**5.** Pharmazeutische Zusammensetzung nach einem der Ansprüche 1 bis 4, wobei eine Anzahl von Aminosäureresten, die eine kationische Gruppe aufweisen und frei von der substituierten Phenylboronsäuregruppe sind, und eine Anzahl von den substituierten Phenylboronsäuregruppen in dem Polyaminosäurekettensegment das folgende Verhältnis erfüllen.

[Math. 1]

$$\sqrt{\begin{array}{c}\text{Anzahl von kationischen}\\ \text{Aminosäureresten frei von}\\ \text{substituierter Phenylboronsäuregruppe in}\\ \text{Polyaminosäurekettensegment}\end{array}} + 2\sqrt{\begin{array}{c}\text{Anzahl von substituierten}\\ \text{Phenylboronsäuregruppen in}\\ \text{Polyaminosäurekettensegment}\end{array}} \geq 12{,}0$$

**6.** Pharmazeutische Zusammensetzung nach einem der Ansprüche 1 bis 5, wobei das Polyaminosäurekettensegment weiter einen Aminosäurerest mit einer hydrophoben Gruppe in einer Seitenkette enthält.

**7.** Blockcopolymer, umfassend:

ein Polyaminosäurekettensegment; und
ein hydrophiles Polymerkettensegment,
wobei:

das Polyaminosäurekettensegment einen Aminosäurerest mit einer kationischen Gruppe in einer Seitenkette und einen Aminosäurerest mit einer substituierten Phenylboronsäuregruppe in einer Seitenkette enthält, wobei die substituierte Phenylboronsäuregruppe mindestens ein durch ein Halogen oder eine Nitrogruppe substituiertes Wasserstoffatom eines Phenylrings aufweist;
die substituierte Phenylboronsäuregruppe in die Seitenkette des Aminosäurerests durch eine Amidbindung, eine Carbamoylbindung, eine Alkylbindung, eine Etherbindung, eine Esterbindung, eine Thioesterbindung, eine Thioetherbindung, eine Sulfonamidbindung, eine Urethanbildung, eine Sulfonylbindung, eine Thyminbindung, eine Harnstoffbindung oder eine Thioharnstoffbindung eingeführt ist;
die substituierte Phenylboronsäuregruppe einen pKa von weniger als 7,5 aufweist; und
eine Anzahl von Aminosäureresten, die eine kationische Gruppe aufweisen und frei von der substituierten Phenylboronsäuregruppe sind, und eine Anzahl von den substituierten Phenylboronsäuregruppen in dem Polyaminosäurekettensegment das folgende Verhältnis erfüllen.

[Math. 2]

$$\sqrt{\begin{array}{c}\text{Anzahl von kationischen}\\ \text{Aminosäureresten frei von}\\ \text{substituierter Phenylboronsäuregruppe in}\\ \text{Polyaminosäurekettensegment}\end{array}} + 2\sqrt{\begin{array}{c}\text{Anzahl von substituierten}\\ \text{Phenylboronsäuregruppen in}\\ \text{Polyaminosäurekettensegment}\end{array}} \geq 12{,}0$$

## Revendications

**1.** Composition pharmaceutique comprenant un complexe de :

un copolymère séquencé comprenant un segment de chaine poly(acide aminé) et un segment de chaine polymère hydrophile, et

un acide nucléique,
où le segment de chaine poly(acide aminé) comprend un résidu acide aminé ayant un groupe cationique dans une chaine latérale et un résidu acide aminé ayant un groupe acide phénylboronique substitué dans une chaine latérale, le groupe acide phénylboronique substitué ayant un cycle phényle dans lequel au moins un atome d'hydrogène est substitué par un halogène ou un groupe nitro ;
le groupe acide phénylboronique substitué est introduit dans la chaine latérale du résidu acide aminé via une liaison amide, une liaison carbamoyle, une liaison alkyle, une liaison éther, une liaison ester, une liaison thioester, une liaison thioéther, une liaison sulfonamide, une liaison uréthanne, une liaison sulfonyle, une liaison thymine, une liaison urée ou une liaison thiourée ;
le groupe acide phénylboronique substitué a un pKa inférieur à 7,5, et
l'acide nucléique forme une liaison covalente réversible avec le groupe acide phénylboronique substitué.

**2.** Composition pharmaceutique selon la revendication 1, où le groupe acide phénylboronique substitué comprend un groupe acide phénylboronique fluoré représenté par la formule (I) suivante :

[Chem. 1]

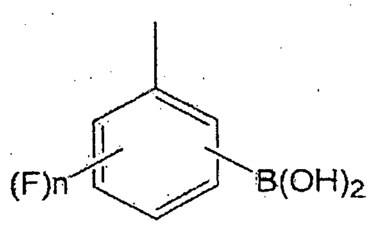

(I)

dans la formule (I) : les F sont présents indépendamment ; n représente 1, 2, 3 ou 4 ; et si n est 1, F et $B(OH)_2$ peuvent être introduits en positions ortho, méta ou para.

**3.** Composition pharmaceutique selon la revendication 1 ou 2, où le groupe cationique comprend un groupe amino.

**4.** Composition pharmaceutique selon l'une quelconque des revendications 1 à 3, où le résidu acide aminé ayant un groupe cationique dans une chaine latérale est un résidu lysine ou un résidu acide aminé obtenu par substitution d'une partie -OH dans un groupe carboxyle (-C(=O)OH) d'un acide aminé acide par l'un quelconque des groupes des formules (i) à (iv) suivantes :

- $NH-(CH_2)_{p1}-[NH-(CH_2)_{q1}-]_{r1}NH_2$ (i);
- $NH-(CH_2)_{p2}-N[-(CH_2)_{q2}-NH_2]_2$(ii);
- $NH-(CH_2)_{p3}-N\{[-(CH_2)_{q3}-NH_2][-(CH_2)_{q4}-NH-]_{r2}H\}$ (iii); et
- $NH-(CH_2)_{p4}-N\{-(CH_2)_{q5}-N[-(CH_2)_{q6}-NH_2]_2\}_2$ (iv)

dans les formules (i) à (iv), p1 à p4, q1 à q6 et r1 et r2 représentent chacun indépendamment, un entier allant de 1 à 5.

**5.** Composition pharmaceutique selon l'une quelconque des revendications 1 à 4, où le nombre de résidus acide aminé qui ont un groupe cationique et qui ne portent pas de groupe acide phénylboronique substitué, et le nombre de groupes acide phénylboronique substitué dans le segment de chaine poly(acide aminé) satisfont la relation suivante.

[Math. 1]

$$\sqrt{\text{Nombre de résidus acide aminé cationiques exempts de groupe acide phényl-boronique substitué dans le segment de chaine poly(acide aminé)}} + 2\sqrt{\text{Nombre de groupes acide phénylboronique dans le segment de chaine poly(acide aminé)}} \geqq 12.0$$

**6.** Composition pharmaceutique selon l'une quelconque des revendications 1 à 5, où le segment de chaine poly(acide aminé) comprend en outre, un résidu acide aminé ayant un groupe hydrophobe dans la chaine latérale.

**7.** Copolymère séquencé comprenant :

un segment de chaine poly(acide aminé), et
un segment de chaine polymère hydrophile,
où le segment de chaine poly(acide aminé) comprend un résidu acide aminé ayant un groupe cationique dans une chaine latérale et un résidu acide aminé ayant un groupe acide phénylboronique substitué dans une chaine latérale, le groupe acide phénylboronique substitué ayant au moins un atome d'hydrogène du cycle phényle substitué par un halogène ou un groupe nitro ;
le groupe acide phénylboronique substitué est introduit dans la chaine latérale du résidu acide aminé via une liaison amide, une liaison carbamoyle, une liaison alkyle, une liaison éther, une liaison ester, une liaison thioester, une liaison thioéther, une liaison sulfonamide, une liaison uréthanne, une liaison sulfonyle, une liaison thymine, une liaison urée ou une liaison thiourée ;
le groupe acide phénylboronique substitué a un pKa inférieur à 7,5 ; et
le nombre de résidus acide aminé, qui ont un groupe cationique et qui ne portent pas de groupe acide phénylboronique substitué, et le nombre de groupes acide phénylboronique substitué dans le segment de chaine poly(acide aminé) satisfont la relation suivante.

[Math. 2]

$$\sqrt{\text{Nombre de résidus acide aminé cationiques exempts de groupe acide phényl-boronique substitué dans le segment de chaine poly(acide aminé)}} + 2\sqrt{\text{Nombre de groupes acide phénylboronique dans le segment de chaine poly(acide aminé)}} \geqq 12.0$$

FIG. 1

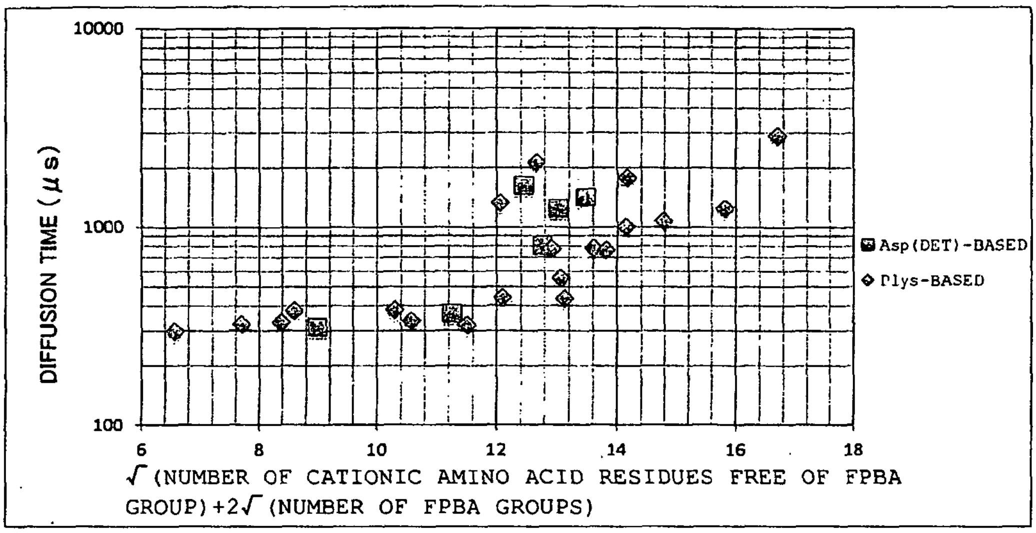
10000
1000
100
DIFFUSION TIME (μs)
6
8
10
12
14
16
18
Asp(DET)-BASED
Plys-BASED
√(NUMBER OF CATIONIC AMINO ACID RESIDUES FREE OF FPBA GROUP)+2√(NUMBER OF FPBA GROUPS)

FIG. 2

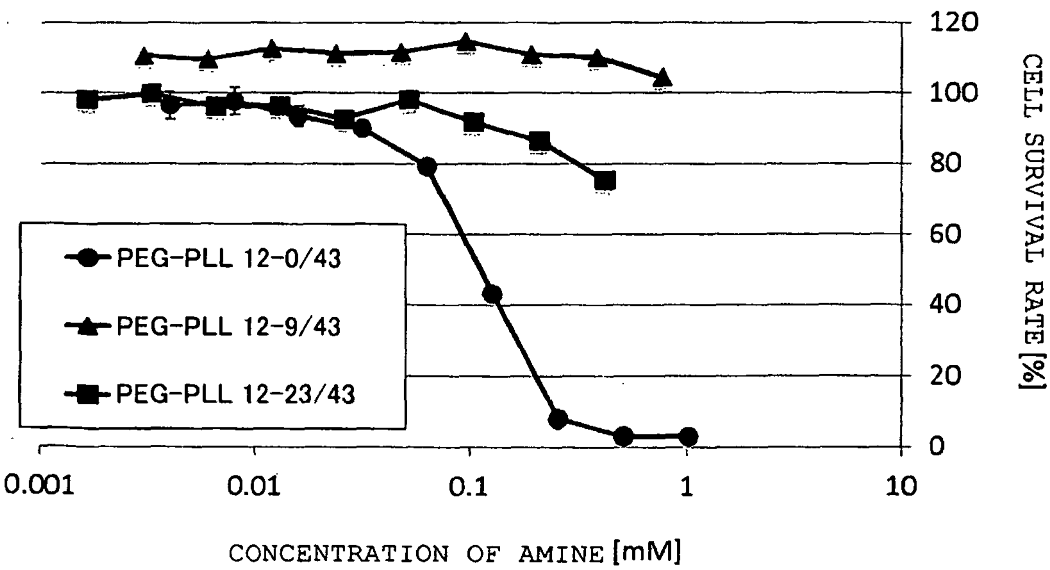
PEG-PLL 12-0/43
PEG-PLL 12-9/43
PEG-PLL 12-23/43
120
100
80
60
40
20
0
CELL SURVIVAL RATE [%]
0.001
0.01
0.1
1
10
CONCENTRATION OF AMINE [mM]

FIG. 3

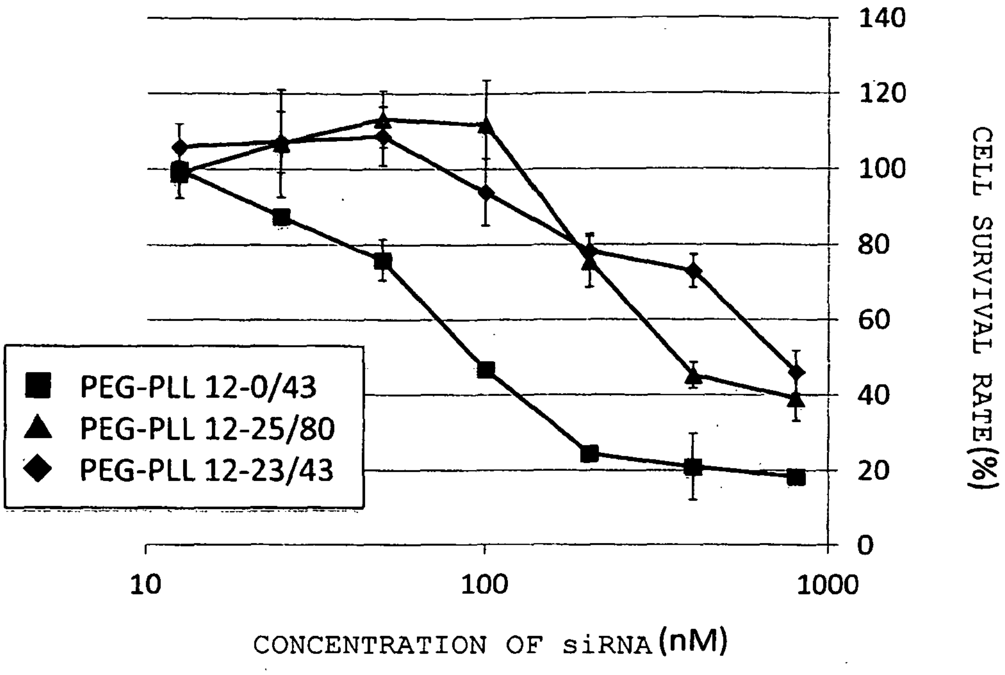
140
120
100
80
60
40
20
0
CELL SURVIVAL RATE(%)
PEG-PLL 12-0/43
PEG-PLL 12-25/80
PEG-PLL 12-23/43
10
100
1000
CONCENTRATION OF siRNA(nM)

FIG. 4

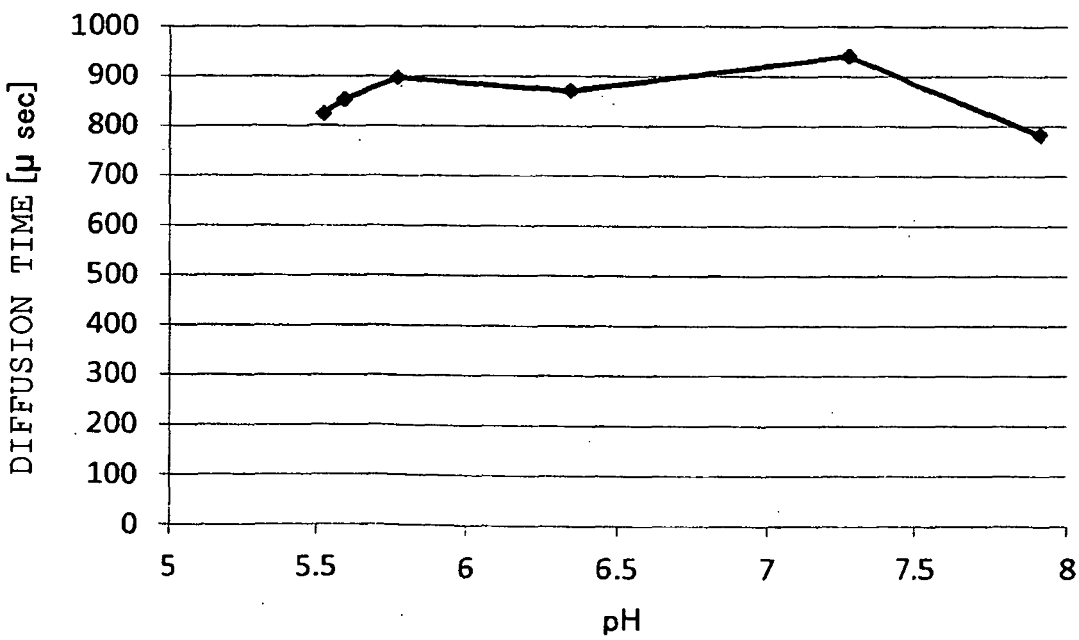
1000
900
800
700
600
500
400
300
200
100
0
DIFFUSION TIME [μ sec]
5
5.5
6
6.5
7
7.5
8
pH

FIG. 5

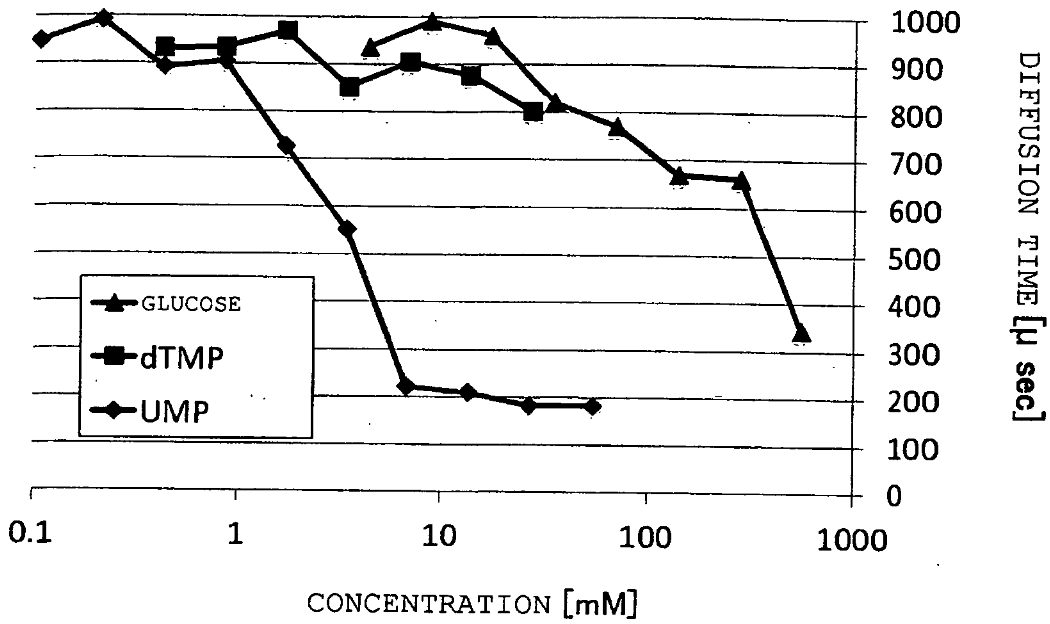
GLUCOSE
dTMP
UMP
1000
900
800
700
600
500
400
300
200
100
0
DIFFUSION TIME [μ sec]
0.1
1
10
100
1000
CONCENTRATION [mM]

FIG. 6

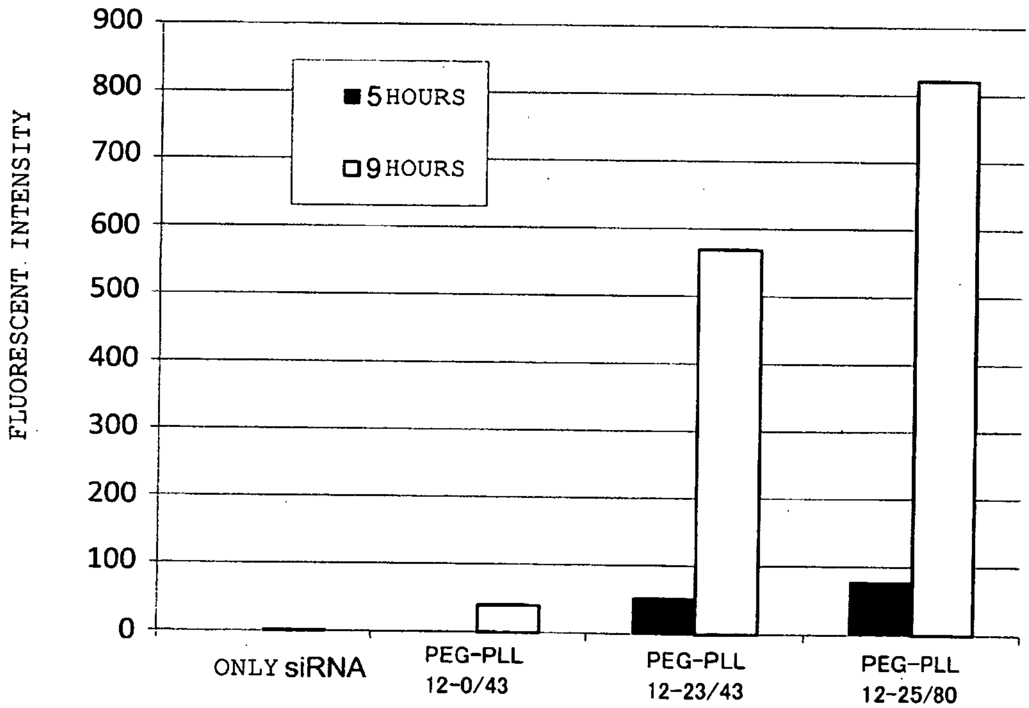
900
800
700
600
500
400
300
200
100
0
FLUORESCENT INTENSITY
5 HOURS
9 HOURS
ONLY siRNA
PEG-PLL 12-0/43
PEG-PLL 12-23/43
PEG-PLL 12-25/80

FIG. 7

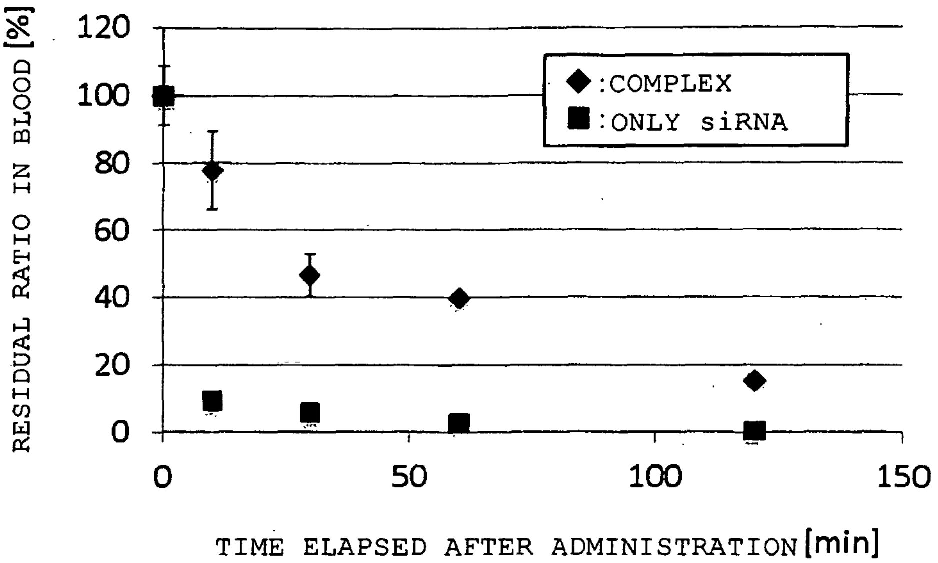
RESIDUAL RATIO IN BLOOD [%]
120
100
80
60
40
20
0
◆:COMPLEX
■:ONLY siRNA
0
50
100
150
TIME ELAPSED AFTER ADMINISTRATION [min]

FIG. 8

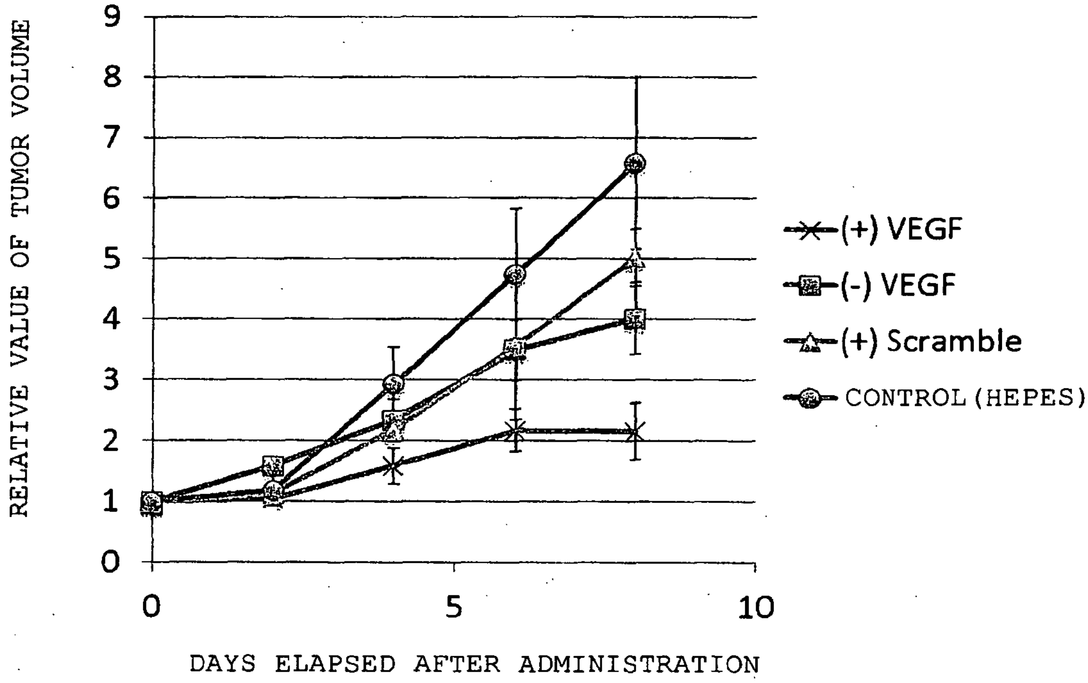
RELATIVE VALUE OF TUMOR VOLUME
9
8
7
6
5
4
3
2
1
0
(+) VEGF
(-) VEGF
(+) Scramble
CONTROL (HEPES)
0
5
10
DAYS ELAPSED AFTER ADMINISTRATION

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2011140537 A
- JP 2002179683 A
- EP 12850688 A
- US 61561022 B